# EUROPEAN PATENT APPLICATION

(11) **EP 4 552 679 A1**
(43) Date of publication of application: **14.05.2025**
(21) Application number: 24211431.2
(22) Date of filing: 07.11.2024
(51) Int. Cl.: A61M 25/00, A61M 25/01, A61B 17/22, A61B 17/221

(54) **CATHETER INCLUDING A RADIOPAQUE OR RADIOGRAPHIC PORTION**

(30) Priority: 08.11.2023 GR 20230100928; 18.10.2024 US 202418919595
(71) Applicant: Covidien LP, Mansfield, MA 02048 (US)
(72) Inventor: MINTZ, Eric Corey, Mansfield MA 02048 (US); WANG, Bin, Mansfield MA 02048 (US); TOURIS, Athanasios, Mansfield MA 02048 (US); EHSANIPOUR, Arshia Amin, Mansfield MA 02048 (US)
(74) Representative: Gray, James

(57) **Abstract**

A catheter includes an elongated body including a distal body portion (60) and defining a body inner lumen. The catheter includes an expandable member (20) coupled to and extending from the distal body portion. The expandable member includes an expandable support member (70) and a polymer jacket (48). The expandable member defines an expandable member inner lumen. The expandable member inner lumen is in fluid communication with the body inner lumen. The expandable support member includes a tubular body and is configured to self-expand to expand the expandable member inner lumen radially outward. The polymer jacket at least partially overlays the expandable support member. The polymer jacket includes polymer loaded with a radiographic material, and the radiographic material configured to be visible via medical imaging.

## Description

### TECHNICAL FIELD

This disclosure relates to a medical catheter.

### BACKGROUND

A medical catheter defining at least one lumen has been proposed for use with various medical procedures. For example, in some cases, a medical catheter may be used to access and treat defects in blood vessels, such as, but not limited to, lesions or occlusions in blood vessels.

### SUMMARY

This disclosure describes example catheters including an elongated body and an expandable member at a distal portion of the elongated body and defining at least part of a distal tip of the catheter. The expandable member is configured to expand radially outward within a hollow anatomical structure (e.g., a blood vessel) of a patient, such as to engage a thrombus. The expandable member is formed from materials (e.g., polymers loaded or compounded with radiopaque materials) that enable a distal portion of the catheter to be radiographic and/or radiopaque without the addition of a separate radiopaque marker (e.g., a solid metal ring of radiopaque material separate from and connected to the elongated body) at the distal portion of the catheter. A solid metal radiopaque marker band may contribute to the overall stiffness of a distal tip of a catheter. Forming the expandable member with a radiopaque material may enable the solid metal radiopaque marker to be eliminated from the distal tip of the catheter, thereby enabling the distal tip of the catheter to be more flexible. In addition, because the expandable member includes a polymer loaded with a radiopaque material, the expandable member may exhibit better radiopacity as compared to using a separate radiopaque structure (e.g., a radiopaque braid, coil, or mesh) incorporated into the expandable member. Using polymers loaded with radiopaque materials may also simplify assembly and provide a lower-cost alternative to radiopaque braids, coils, or meshes. Additionally, polymers loaded with radiopaque materials may provide a clinician with a better indication of a location of the distal portion within a patient.

The combination of materials described in this disclosure may enable a relatively high loading concentration of radiopaque materials without compromising the physical integrity of the portions loaded with radiopaque materials. In some catheters with a structure including polymers loaded with radiopaque materials (e.g., tungsten), degradation via hydrolysis may lead to shorter shelf life, lead to limited processing windows during manufacturing (e.g., exposure to heat), or necessitate the use of higher durometer polymers, which may otherwise decrease the flexibility of the structure. According to the examples of this disclosure, the expandable members can include one or more materials selected to reduce or inhibit degradation (e.g., via hydrolysis) of the structure of the expandable members, while simultaneously enabling a relative high loading concentration of radiopaque materials. In some examples, a base polymer used for the expandable members which is loaded with radiographic material includes a Styrenic Block Copolymers (SBC). Further, the one or more materials loaded with radiopaque material may include modifications (e.g., via grafting) to facilitate adhesion and/or bonding to other components of the catheter.

In some examples, a catheter includes an elongated body including a distal body portion and defining a body inner lumen. The catheter includes an expandable member coupled to and extending from the distal body portion. The expandable member includes an expandable support member and a polymer jacket. The expandable member defines an expandable member inner lumen. The expandable member inner lumen is in fluid communication with the body inner lumen. The expandable support member includes a tubular body and is configured to self-expand to expand the expandable member inner lumen radially outward. The polymer jacket at least partially overlays the expandable support member. The polymer jacket includes polymer loaded with a radiographic material, and the radiographic material configured to be visible via medical imaging.

In some examples, a catheter includes an elongated body including a distal body portion and defining a body inner lumen. The catheter includes a polymer jacket located at the distal body portion. The polymer jacket includes a polymer loaded with a radiographic material configured to be visible via medical imaging. The polymer includes a Styrenic Block Copolymer (SBC).

In some examples, a method includes positioning a polymer jacket including a radiographic material over a portion of an expandable support member of a catheter. In some examples, the method further includes reflowing the polymer jacket onto the catheter. When assembled, the catheter includes an elongated body including a distal body portion and defining a body inner lumen. When assembled, the catheter includes an expandable member coupled to and extending from the distal body portion. The expandable member includes the expandable support member and the polymer jacket. The expandable member defines an expandable member inner lumen, wherein the expandable member inner lumen is in fluid communication with the body inner lumen. The expandable support member includes a tubular body and is configured to self-expand to expand the expandable member inner lumen radially outward. The polymer jacket at least partially overlays the expandable support member and includes a polymer loaded with a radiographic material, and the radiographic material configured to be visible via medical imaging.

This disclosure also describes examples of methods using the catheters.

The examples described herein may be combined in any permutation or combination.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

FIG. 1 is a conceptual side view of an example catheter, which includes an elongated body and an expandable member at a distal portion of the elongated body.
FIG. 2 is a conceptual cross-sectional view of an example of the distal portion of the catheter of FIG. 1, including the expandable member, where the cross-section is taken through a center of the catheter and along a longitudinal axis.
FIG. 3 is a conceptual cross-sectional view of another example of the distal portion of the catheter of FIG. 1, where the cross-section is taken through a center of the catheter and along a longitudinal axis.
FIG. 4 is a conceptual cross-sectional view of another example of the distal portion of the catheter of FIG. 1, where the cross-section is taken through a center of the catheter and along a longitudinal axis.
FIG. 5 is a conceptual cross-sectional view of another example of the distal portion of the catheter of FIG. 1, where the cross-section is taken through a center of the catheter and along a longitudinal axis.
FIG. 6 is a conceptual view of an expandable member of the catheter of FIG. 1.
FIGS. 7A and 7B are conceptual cross-sectional side views of an example catheter, which includes an introducer sheath.
FIG. 8 is a flow diagram of an example method of using a catheter.
FIG. 9 is a flow diagram of an example method of forming a catheter.

### DETAILED DESCRIPTION

The disclosure describes a medical device, referred to herein as a "catheter," including an expandable member configured to expand radially outward within a hollow anatomical structure (e.g., a blood vessel) of a patient, e.g., to engage with a thrombus to facilitate aspiration of the thrombus (or other material or object(s) to be removed, such as a plaque or foreign body). Some catheters include a distinct radiopaque marker band (e.g., a solid metal ring) near a distal tip of the catheter, which may facilitate placement of the catheter via fluoroscopic imaging. This solid metal radiopaque marker band may also contribute to an overall (e.g., excessive) stiffness of the distal tip of the catheter, which may adversely impact navigability of the catheter to distal sites in the vasculature of a patient. Additionally or alternatively, some catheters use radiopaque braids or other radiopaque mesh structures, which may give some visibility via suitable medical imaging techniques (e.g., fluoroscopic imaging).

According to the examples of this disclosure, the expandable members described herein can be integrally formed (e.g., loaded and or compounded) with one or more materials that enable a distal tip or portion of the catheter to be radiographic or radiopaque without the addition of a separate, more-rigid radiopaque marker band (e.g., a solid metal radiopaque marker band), thereby improving the flexibility of the distal tip and improving the navigability of the catheter. In addition, because a length of the expandable member is formed from a radiopaque material as compared to a separate, smaller or shorter (as measured in a direction parallel to a longitudinal axis of the catheter) radiopaque marker band, a longer portion of the distal tip or distal portion of the catheter (e.g., about 1 centimeter (cm) to about 10 cm) may be visible under fluoroscopy or x-ray imaging, thereby providing a clinician with a better indication of a location of the distal tip or distal portion within the vasculature of the patient. Further, because the expandable members according to the examples of this disclosure are loaded with radiographic materials (e.g., tungsten), the radiopacity and/or visibility of the of the expandable members may be greater as compared to other expandable members that use radiopaque braids or radiopaque mesh structures. Using polymers loaded with radiopaque materials may also simplify assembly by eliminating the need to separate processes to incorporate radiopaque braids, coils, or meshes.

Example catheters in accordance with this disclosure include a relatively flexible elongated body configured to be navigated through vasculature of a patient, e.g., tortuous vasculature in a brain of the patient. The elongated body may include a plurality of concentric layers, such as one or more inner liners, one or more outer jackets, and a structural support member (e.g., a coil, braid, and/or hypotube) positioned between at least a portion of the one or more inner liners and the one or more outer jackets. A distal tip or distal portion of the catheter includes an expandable member, such as an expandable stent-like structure or an expandable braid, positioned distal to a distal portion of the elongated body. In some examples, the expandable member is distinct from, but mechanically coupled to, the distal portion of the elongated body. In other examples, the expandable member is integrally formed with (e.g., laminated with and/or forming a distal extension of) the distal portion of the elongated body. The expandable member is configured to expand radially outward within a hollow anatomical structure (e.g., a blood vessel) of the patient. This may enable, for example, the expandable member to engage with a thrombus, such as a clot, embolism, or other material such as plaques or foreign bodies during an aspiration procedure, such as, but not limited to, a medical procedure using A Direct Aspiration First Pass Technique (ADAPT) for acute stroke thrombectomy.

The expandable member may help improve aspiration of the thrombus into the catheter by providing a relatively large luminal diameter (and therefore exerting a larger aspiration force against the thrombus or other material to be removed) and interior space for the thrombus to engage with the catheter compared to examples in which an otherwise similar catheter does not include an expandable member. For example, such a catheter that does not include an expandable member may have limited radial expansion due to a structural support member that extends to the distal end of the catheter, and may thus make it harder to aspirate a thrombus (e.g., due to a smaller cross-sectional dimension of the distal end of the catheter). The expandable member may overcome such radial expansion limitations, thereby increasing thrombus engagement, reducing the amount of time required for revascularization, and increasing revascularization success rates for various procedures, as compared to similar procedures performed using catheters that do not include an expandable member to engage a thrombus.

The materials of the catheters, and specifically, the expandable members, according to the examples of this disclosure may enable a relatively high loading concentration of radiopaque materials without compromising the physical integrity of the portions loaded with radiopaque materials. In some examples, materials (e.g., polymers) be selected to reduce or inhibit degradation. In some catheters with a structure including polymers loaded with radiopaque materials (e.g., tungsten), degradation of the structure may lead to shorter shelf life, limited processing windows during manufacturing (e.g., exposure to heat), or necessitate the use of higher durometer polymers, which may otherwise decrease the flexibility of the structure. For example, certain radiographic materials (e.g., tungsten) may cause (e.g., act to catalyze) degradation when polymers are loaded with these radiographic materials. According to the examples of this disclosure, the expandable members can include one or more materials selected to reduce or inhibit degradation (e.g., via hydrolysis) of the structure of the expandable members, while simultaneously enabling a relative high loading concentration of radiopaque materials. In some examples, a base polymer used for the expandable members which is loaded with radiographic material includes a Styrenic Block Copolymer (SBC). Further, the one or more materials may include modifications (e.g., via grafting or other functionalization) to facilitate adhesion and/or bonding to other components of the catheter.

While the devices, systems, and methods described herein are primarily described in the context of aspiration within the neurovasculature, it should be appreciated that the techniques of this disclosure are applicable to other elongated bodies configured to be inserted into a body cavity. For example, loading polymers with radiographic and/or radiopaque materials for visualization under a suitable medical imaging technique may be used in elongated bodies that do not use expandable members. Further, loading polymers with radiographic and/or radiopaque materials for visualization under a suitable medical imaging technique may be applicable to other medical therapies (e.g., ablation, angioplasty, drug delivery, delivery of implantable medical devices, nerve stimulation such as spinal cord stimulation, deep brain stimulation, sacral neuromodulation, etc.) and other target anatomy (e.g., the heart, non-vascular brain tissue, peripheral vasculature, the gastrointestinal tract, etc.).

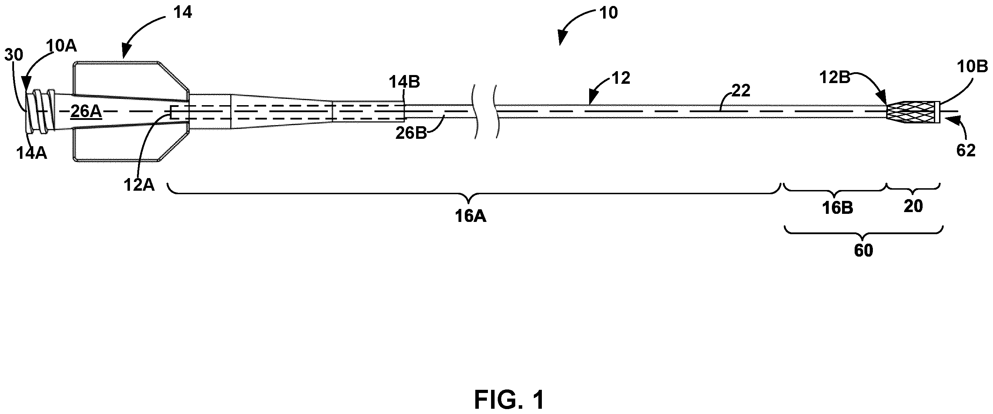

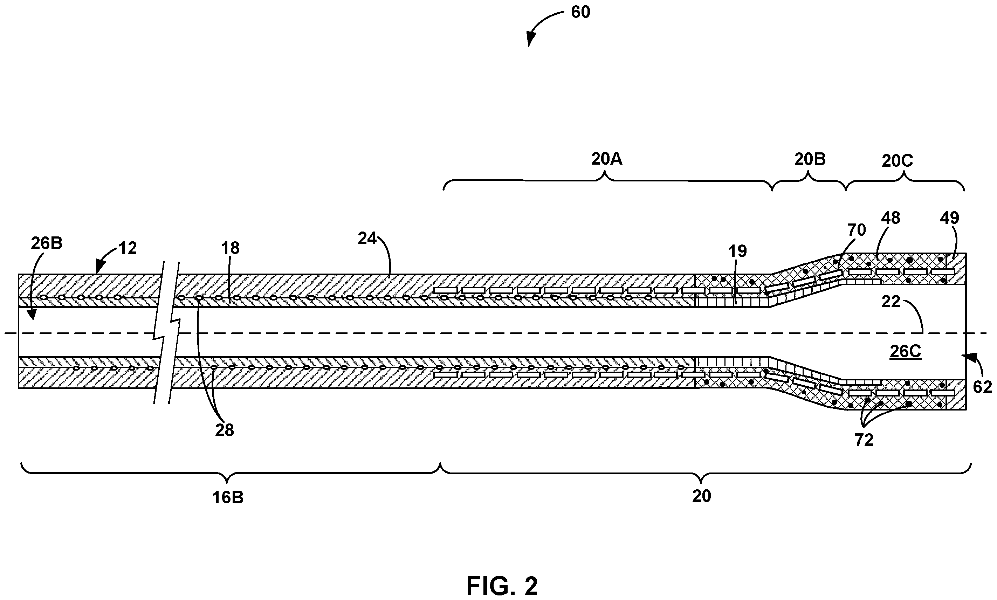

FIG. 1 is a conceptual side view of an example catheter 10, and FIG. 2 is a conceptual cross-sectional side view of a distal tip or distal portion 60 of the example catheter 10, where the cross-section is taken through a center of distal portion 60 along a longitudinal axis 22. As shown in FIGS. 1 and 2, catheter 10 can include an elongated body 12, a hub 14, and an expandable member 20. Catheter 10 defines an inner lumen 26, which is shown in the examples of FIGS. 1 and 2 as including a hub lumen 26A, a body lumen 26B (which may also be referred to herein as body inner lumen 26B), and an expandable member lumen 26C.

Elongated body 12 is configured to be advanced through vasculature of a patient via a pushing force applied to proximal body portion 16A (e.g., via hub 14) of elongated body 12 without buckling, kinking, or otherwise undesirably deforming (e.g., ovalization). As shown in FIG. 2, elongated body 12 can include a plurality of concentric layers, such as a first inner liner 18 (which may also be referred to herein as body inner liner 18), a first outer jacket 24 (which may also be referred to herein as body outer jacket 24, body polymer jacket 24, or outer jacket 24), and a structural support member 28 positioned between at least a portion of inner liner 18 and at least a portion of outer jacket 24. As shown in FIG. 2, elongated body 12 can also include a second inner liner 19 distal to the first inner liner 18 and a second outer jacket 48 (which may also be referred to herein as polymer jacket 48 or outer jacket 48) distal to the first outer jacket 24. In some examples, elongated body 12 additionally includes a third outer jacket 49 (which may also be referred to herein as distal tip polymer jacket 49, tip outer jacket 49, or outer jacket 49) distal to polymer jacket 48. In some examples, elongated body 12 includes one or more tie layers (not shown) between first inner liner 18 and body outer jacket 24 and/or between second inner liner 19 and outer jacket 48. Elongated body 12 includes a proximal body portion 16A and a distal body portion 16B, which are each longitudinal sections of elongated body 12 and do not overlap in the longitudinal direction (along longitudinal axis 22). Elongated body 12 extends from a body proximal end 12A to a body distal end 12B and defines at least one body lumen 26B. In the example shown in FIG. 1, proximal end 12A of elongated body 12 is received within hub 14 and is mechanically connected to hub 14 via an adhesive, welding, or another suitable technique or combination of techniques. Inner lumen 26 of catheter 10 may be defined by portions of hub 14, portions of elongated body 12, including inner liner 18, and portions of expandable member 20, including inner liner 19.

Catheter 10 may be used as an aspiration catheter to remove a thrombus or other material such as plaques or foreign bodies from vasculature of a patient. In such examples, a suction force (e.g., a vacuum) may be applied to proximal end 10A of catheter 10 (e.g., via hub 14) to draw the thrombus or other blockage into inner lumen 26. An aspiration catheter may be used in various medical procedures, such as a medical procedure to treat an ischemic insult, which may occur due to occlusion of a blood vessel (arterial or venous) that deprives brain tissue, heart tissue or other tissues of oxygen-carrying blood.

In some examples, catheter 10 is configured to access relatively distal locations in a patient including, for example, the middle cerebral artery (MCA), internal carotid artery (ICA), the Circle of Willis, and tissue sites more distal than the MCA, ICA, and the Circle of Willis. The MCA, as well as other vasculature in the brain or other relatively distal tissue sites (e.g., relative to the vascular access point), may be relatively difficult to reach with a catheter, due at least in part to the tortuous pathway (e.g., comprising relatively sharp twists or turns) through the vasculature to reach these tissue sites. Elongated body 12 may be structurally configured to be relatively flexible, pushable, and relatively kink- and buckle- resistant, so that it may resist buckling when a pushing force is applied to a relatively proximal section of catheter 10 (e.g., via hub 14) to advance elongated body 12 distally through vasculature, and so that it may resist kinking when traversing around a tight turn in the vasculature. In some examples, elongated body 12 is configured to substantially conform to the curvature of the vasculature. In addition, in some examples, elongated body 12 has a column strength and flexibility that allow at least distal body portion 16B of elongated body 12 to be navigated from a femoral artery, through the aorta of the patient, and into the intracranial vascular system of the patient, e.g., to reach a relatively distal treatment site.

Although primarily described as being used to reach relatively distal vasculature sites, catheter 10 may also be configured to be used with other target tissue sites. For example, catheter 10 may be used to access tissue sites throughout the coronary and peripheral vasculature, the gastrointestinal tract, the urethra, ureters, fallopian tubes, veins and other hollow anatomical structures of a patient.

In some examples, a "working length" of catheter 10 may be measured from distal end 14B of hub 14 (e.g., a distal end of a strain relief member of a hub assembly) to distal end 10B of catheter 10 along longitudinal axis 22. The working length of catheter 10 may depend on the location of the target tissue site within the body of a patient or may depend on the medical procedure for which catheter 10 is used. For example, if catheter 10 is a distal access catheter used to access vasculature in a brain of a patient from a femoral artery access point at the groin of the patient, catheter 10 may have a working length of about 115 centimeters (cm) (e.g., to account for slight differences in length, such as due to manufacturing tolerances) to about 145 cm (e.g., to account for slight differences in length, such as due to manufacturing tolerances) or more, such as about 130 cm, although other lengths may be used. The distal tip or distal portion 60 of catheter 10, including distal body portion 16B of elongated body 12 and expandable member 20, may be about 5 cm (e.g., to account for slight differences in length, such as due to manufacturing tolerances) to about 35 cm in length (e.g., to account for slight differences in length, such as due to manufacturing tolerances). Proximal body portion 16A of elongated body 12 may be about 90 cm (e.g., to account for slight differences in length, such as due to manufacturing tolerances) to about 130 cm in length (e.g., to account for slight differences in length, such as due to manufacturing tolerances), depending on the length of distal tip or distal portion 60.

Expandable member 20 is configured to radially expand within a vessel of a patient, e.g., to engage a thrombus within the vessel. As shown in FIG. 2, expandable member 20, in the deployed (e.g., expanded) configuration, can have a proximal section 20A, a tapering section 20B, and a distal section 20C. Expandable member 20 is positioned at (e.g., overlapping with or entirely distal to) distal body portion 16B of elongated body 12, such that a distal end of expandable member 20 defines distal end 10B of catheter 10 and a distal mouth 62 open to inner lumen 26 of catheter 10. For example, expandable member lumen 26C (also referred to as expandable member inner lumen 26C) forms a distal extension of body inner lumen 26B of the elongated body 12. In these examples, expandable member lumen 26C is in fluid communication with body inner lumen 26B of the elongated body 12.

In some examples, each of proximal section 20A, tapering section 20B, and distal section 20C define different diameters when expandable member 20 is in the deployed (e.g., expanded) configuration. In some examples, proximal section 20A defines an inner diameter and/or outer diameter that is substantially equal to the inner and/or outer diameter(s) of distal portion 16B of elongated body 12, even when expandable member 20 is in the deployed configuration as shown in FIG. 2. In some examples, distal section 20C (e.g., a distal-most section of expandable member 20), when in the deployed (e.g., expanded) configuration, defines a larger inner diameter and outer diameter than distal portion 16B of elongated body 12. Distal section 20C can be configured to be generally cylindrical, with a constant or substantially constant inner diameter and/or outer diameter along its length. The length of distal section 20C can be 0.5 cm to about 3 cm, or 0.5 cm to about 2.5 cm, to facilitate engulfing a thrombus during use (without being so long as to generate unacceptable levels of friction during delivery to a patient through a surrounding catheter or sheath). Expandable member 20 (e.g., distal section 20C thereof) can be configured to be self-expanding, e.g., upon advancement beyond the end of a surrounding catheter.

In some examples, expandable member 20 includes an expandable support member 70 configured to expand radially outward, thereby expanding expandable member lumen 26C radially outward. For example, expandable support member 70 can enable expandable member 20 to maintain its expanded shape (after it is expanded), even in the presence of a suction force applied to inner lumen 26 of catheter 10 during an aspiration process. In general, expandable support member 70 can provide structurally integrity to expandable support member 20 (e.g., to resist deformation in the presence of force). In some examples, expandable support member 70 includes braided structure, a frame, an expandable stent-like structure, or the like, which can each be formed from a plurality of structural elements. In some examples, expandable support member 70 includes a braided structure comprising interwoven filaments, each filament being formed from such a structural element. In some examples, expandable support member 70 of expandable member 20 may be formed from radiopaque structural elements. In some examples, a proximalmost end of expandable member 20 corresponds to a proximalmost end of expandable support member 70, and/or a distalmost end of expandable member 20 corresponds to distal mouth 62.

The inside diameter and/or outside diameter of distal section 20C (in the deployed configuration) can be established by heat-setting expandable support member 70 on a generally cylindrical mandrel having a mandrel diameter approximately equal to the desired expanded-configuration inside diameter of expandable member 20. In this manner, the expanded-configuration inside and/or outside diameter of distal section 20C can be selected to enable distal section 20C to make firm contact with the vessel wall when expanded, and provide a large distal mouth 62 for application of high suction force to a thrombus or other material to be aspirated. However, it can also be desirable not to allow the expanded-configuration inside and/or outside diameter of distal section 20C to become too large, as this can make it difficult to advance catheter 10 through a surrounding catheter or sheath during insertion into a patient (as an aggressively expansive expandable member 20 generates high friction forces against the inner wall of the surrounding catheter or sheath). Consequently, the expanded-configuration outside diameter of distal section 20C can be about 150 percent to about 300 percent of the outside diameter of distal portion 16B of elongated body 12 (or of the outside diameter of proximal section 20A of expandable member 20). In some examples, the expanded-configuration outer diameter of distal section 20C can be about 110 percent, 120 percent, 150 percent, 200 percent, 250 percent, or 300 percent of the outside diameter of distal portion 16B of elongated body 12 (or of the outside diameter of the proximal end of expandable member 20). In some examples, an outer diameter of the distal section 20C (e.g., a cylindrical tube) is no more than 300 percent of the outer diameter of the distal body portion 16B.

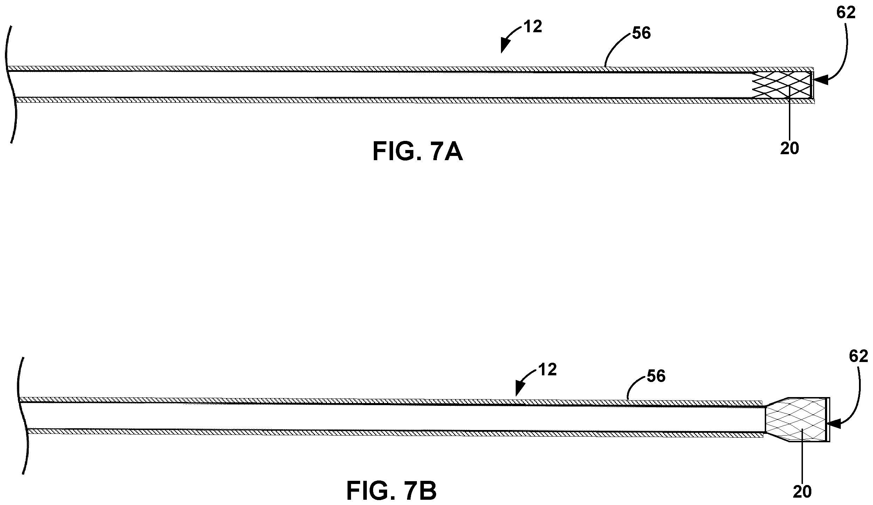

In some examples, expandable member 20 includes outer jacket 48 coupled to (e.g., radially inward and/or radially outward of) and overlays (e.g., at least partially overlays or fully overlays) expandable support member 70, or integrated into expandable support member 70. In some examples, outer jacket 48 is formed of an elastomeric material that permits the expansion of expandable member 20 to a deployed (e.g., expanded configuration) and collapse to a delivery (e.g., compressed) configuration. In some examples, polymer jacket 48 of expandable member 20 is configured to be compressible and have a relatively low flexural stiffness to allow for easy bending, as well to allow for expandable member 20 to be compressed into a delivery configuration (e.g., to fit into a delivery sheath, as discussed with respect to FIGS. 7A and 7B). In some examples, outer jacket 48 has a shore A hardness of a about 20A to about 80A hardness (inclusive), such as about 30A to 40A hardness (inclusive), about 30A to 60A hardness (inclusive), or about 30A to 80A hardness (inclusive). In some examples, outer jacket 48 includes a fluid-impermeable polymer. The stiffness of outer jacket 48 can be measured by, for example, a flexural stiffness or a torsional stiffness value. In some examples, outer jacket 48 may enable catheter 10 to exhibit a more flexible distal portion (e.g., throughout a majority of expandable member 20) while still retaining sufficient strength and rigidity throughout the majority of elongated body 12 for navigation.

In some examples, outer jacket 48 includes a more flexible (e.g., less stiff) and/or softer (e.g., less hard) material than body outer jacket 24, first inner liner 18, and/or second inner liner 19. In some examples, outer jacket 48 has a lower coefficient of friction and/or a lower modulus of elasticity than body outer jacket 24, first inner liner 18, and/or second inner liner 19.

In some examples, expandable member 20 includes tip outer jacket 49 coupled to (e.g., radially inward and/or radially outward of) expandable support member 70, or integrated into expandable support member 70. In some examples, tip outer jacket 49 is distal to outer jacket 48. In some examples a proximal end (e.g., a proximalmost end) of tip outer jacket 49 abuts (e.g., contacts) a distal end (e.g., a distalmost end) of outer jacket 48. In some examples, tip outer jacket 49 extends to a distalmost end of catheter 10, and/or a distalmost end of elongated body 12. In some examples, tip outer jacket 49 is less flexible than outer jacket 48. In some examples, tip outer jacket 49 includes a material (e.g., a polymer) with a higher shore A hardness than outer jacket 48. In some examples, tip outer jacket 49 has a shore A hardness of 80A or about 80A (e.g., such as to account for slight variations in manufacturing tolerances). In some examples, tip outer jacket 49 has a shore A hardness between about 80A and about 90A (e.g., such as to account for slight variations in manufacturing tolerances). In some examples, tip outer jacket 49 includes the same material as outer jacket 48 (e.g., to facilitate bonding and/or adhesion between tip outer jacket 49 and outer jacket 48), but a different shore A hardness. In other examples, tip outer jacket 49 includes a different material as outer jacket 48. In some examples, an inner liner is not provided that is radially inward of tip outer jacket 49. In the example of FIG. 2, tip outer jacket 49 is distal of a distalmost end of inner liner 18 and a distalmost end of inner liner 19. Further, tip outer jacket 49 may be longitudinally separated by a gap from a distal end of inner liner 18 and/or inner liner 19.

In some examples, tip outer jacket 49 of expandable support member 70 is configured to encapsulate and/or constrain a distal end (e.g., the distalmost end) of expandable support member 70, which may include one or more individual braid wires. For example, the relatively rigid polymer (e.g., as compared to outer jacket 48) of tip outer jacket 49 may help cover the individual braid wires of expandable support member 70 to avoid snagging, tearing, or generally degradation of the distal tip of expandable member 20. In other words, the relatively rigid polymer of tip outer jacket 49 may be configured to constrain the distal end (e.g., the individual braid wires) of expandable support member 70 such that the distal end (e.g., the individual braid wires) is not exposed to the vasculature of the patient (e.g., which may cause undesirable trauma to the vessel wall). In some examples, tip outer jacket 49 includes a longitudinal length sufficient (e.g., as measured along longitudinal axis 22) to encapsulate (e.g., longitudinally overlap with) any exposed ends of the individual braid wires of expandable support member 70. In some examples, tip outer jacket 49 defines a longitudinal length (e.g., measured along longitudinal axis 22) of about 0.25 mm to about 0.75 mm, such as 0.5 mm or about 0.5 mm (e.g., such as to account for slight variations in length, such as due to manufacturing tolerances). In some examples, tip outer jacket 49 extends distally of the distal end (e.g., the distalmost end) of expandable support member 70.

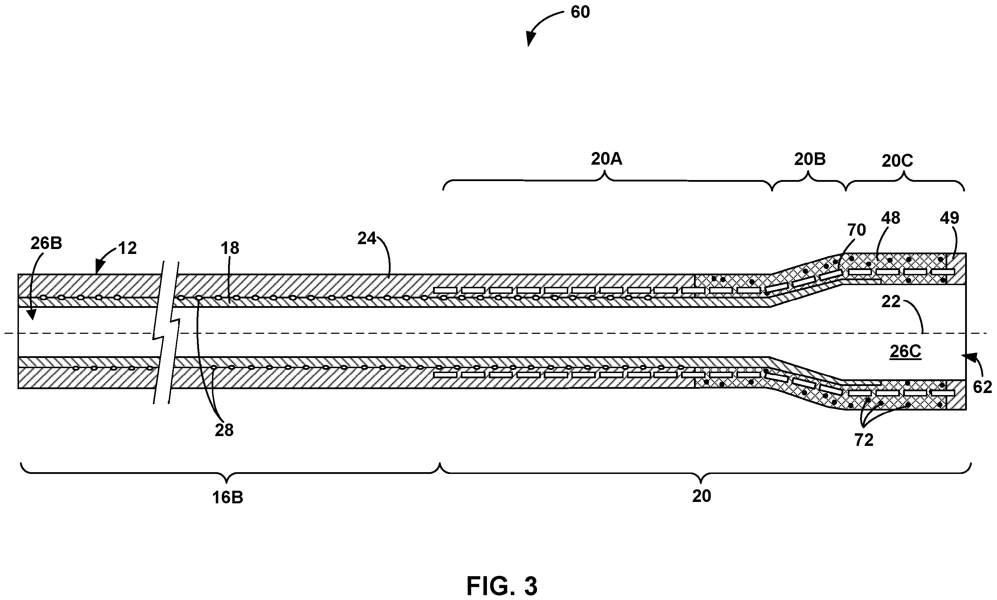

In some examples, elongated body 12 includes inner liner 19 extending along the taper of expandable support member 70 (e.g., at least a portion of the taper of expandable support member 70), which may facilitate encapsulation of expandable support member 70. In some examples, outer jacket 48 may be formed be reflowing a polymer tube over a portion of expandable support member 70 including the taper of expandable support member 70 (e.g., corresponding to tapering section 20B of expandable member 20). Inner liner 19 can facilitate braid encapsulation (e.g., the braid of expandable support member 70) across the taper of expandable support member 70. In some examples, inner liner 19 is configured to be a tie layer between outer jacket 48 and expandable support member 70. As shown in the examiner of FIG. 2, inner liner 19 extends longitudinally across tapering section 20B (e.g., proximally of a proximal end of tapering section 20B and distally of a distal end of tapering section 20B). In some examples, inner liner 19 does not extend does not extend to the distal end of expandable member 20 (e.g., polymer jacket 48 extends distally of inner liner 19). In some examples, expandable support member 70 extends distally of a distalmost end of inner liner 19. In some examples, expandable support member 70 extends proximally of a proximalmost end of inner liner 19. In examples in which a second inner liner 19 is not provided, but inner liner 18 extends across tapering section 20B (e.g., FIG. 3), inner liner 18 may include the properties of inner liner 19 with relation to other components described herein.

In examples described herein, polymer jacket 48 of expandable member 20 includes (e.g., is loaded or compounded with) a radiographic material 72 (also referred to herein as a radiopaque material 72). In examples where polymer jacket 48 of expandable member 20 includes radiographic material 72, a separate marker band (e.g., solid metal marker band) or other radiopaque structure (e.g., expandable support member 70) may not be provided and/or needed. Such configurations enable a solid metal radiopaque marker band to be eliminated from the distal tip of catheter 10 while still enabling the distal tip of catheter 10 to be radiopaque, which may provide one or more advantages over a catheter including a solid radiopaque marker band (e.g., a solid metal radiopaque marker band) at the distal tip. For example, a solid metal radiopaque marker band may be formed from a relatively rigid ring of metal (e.g., platinum-iridium), and may contribute to the overall stiffness of a distal tip or portion of a catheter. Forming expandable member 20 from a solid metal radiopaque material may enable such a stiff radiopaque marker band to be eliminated from the distal tip or portion of catheter 10, thereby enabling the distal tip or portion 60 of catheter 10 to be more flexible. This increased radial flexibility (e.g., range of expandability in a radial direction) may be useful, for example, when a relatively smaller introducer catheter is required for insertion via certain vasculature access sites, such as the radial artery. As one non-limiting example, a radial access sheath may have an inner diameter of about 5 French, as compared to about 6 French for femoral access sheaths. Accordingly, a smaller diameter (or other maximum cross-sectional dimension) catheter 10 may be useful for such applications.

In addition to greater flexibility, by loading polymer jacket 48 with radiographic material 72 instead of (or in addition to) a separate radiographic component (e.g., a radiopaque braid, coil, or mesh, such as expandable support member 70), expandable member 20 may exhibit greater radiopacity and thus greater visibility (e.g., via a suitable medical imaging technique, including fluoroscopic imaging) as compared to instances where expandable member 20 does not include a polymer loaded with a radiographic material. For example, because of a higher density or volume of radiographic material present in expandable member 20 when polymer jacket is loaded/compounded with radiographic material 72, expandable member may be more radiographic (e.g., more visible to a clinician via medical imaging) as compared to examples when only a radiographic or radiopaque braid, coil, or mesh is provided. This greater visibility may enable a clinician to better position catheter 10, including expandable member 20 with the vasculature of a patient, relative to a target location (e.g., a thrombus in the vasculature). Further, as radiopaque braids and/or radiopaque mesh structures can be relatively expensive (e.g., nitinol drawn, filled tubing) and/or difficult to incorporate into catheters during manufacturing, loading the polymers with radiographic material may decrease material and/or manufacturing cost as compared to using radiopaque braids and/or radiopaque mesh structures.

Incorporating radiographic material 72 throughout a portion of a length of expandable member 20 (e.g., in polymer jacket 48) enables a longer extent of the distal tip or portion 60 of catheter 10 (e.g., about 1 cm to about 10 cm, such as about 2 cm to about 6 cm) to be visible under a suitable medical imaging technique, such as fluoroscopy or x-ray imaging. This may provide a clinician with a better indication of a location of the distal tip or portion 60 within a patient. In addition, expandable member 20 formed with radiopaque material 72 may be fluoroscopically illuminated, enabling the clinician to monitor the shape of expandable member 20 as the clinician navigates expandable member 20 through the patient's vasculature. For example, the radiopaque material 72 may enable the clinician to observe when expandable member 20 is bending or not bending around a curve in the patient's vasculature. As another example, the radiopaque material 72 may allow the clinician to observe if expandable member 20 becomes kinked or otherwise deformed in an undesirable manner that may inhibit navigation of catheter 10 through the vasculature. A radiopaque marker band alone, on the other hand, may not enable a shape of expandable member 20 to be visible under fluoroscopy or x-ray imaging.

In addition, by incorporating radiopaque material 72 throughout expandable member 20, a clinician may be able to more-easily discern when the distal mouth 62 of expandable member 20 has come into contact with a thrombus within the vasculature of a patient. For example, the clinician may distally advance expandable member 20 through the vasculature of the patient toward a thrombus. When distal mouth 62 of expandable member 20 contacts the thrombus, the thrombus may form a seal over the distal mouth 62 of expandable member 20. In the presence of a suction force (e.g., via an aspiration pump) applied to inner lumen 26 of catheter 10, such a seal over distal mouth 62 of expandable member 20 may cause expandable member 20 to partially axially contract and/or otherwise change shape along longitudinal axis 22. Expandable member 20 may be configured to partially axially contract and/or otherwise change shape when engaged with a thrombus due to its relative flexibility, e.g., compared to expandable members that include a solid metal radiopaque marker band. Due to the radiopaque material of expandable member 20, the clinician may easily observe this axial contraction of expandable member 20 on a fluoroscopic imaging screen, informing the clinician when catheter 10 has engaged the thrombus. If distal mouth 62 becomes disengaged with the thrombus, the clinician may observe a change in the shape of expandable member 20, such as cessation of axial contraction or return to a previous configuration, thereby indicating to the clinician that a change in position or aspiration conditions may be initiated to reengage the thrombus.

In some examples, in its expanded configuration, expandable member 20 defines a tubular, cylindrical, or funnel shape configured to provide catheter 10 with a relatively large diameter (or other maximum cross-sectional dimension) distal end 10B (compared to, for example, proximal body portion 16A of elongated body 12) and expandable member lumen 26C for better engagement with a thrombus (e.g., clot or embolus). In some examples, the cross-section of expandable member 20 in its expanded configuration may be round (e.g., circular) and the cross-sectional axis may be referred to as a diameter. In some examples, the cross-section may be irregularly shaped, in which case the cross-sectional dimension may be referred to as the major axis (e.g., a longest dimension of the cross-section). In the expanded configuration, the cross-section of expandable member 20 may be wider at a distal end than a proximal end. For example, in the expanded configuration, the inner dimension (e.g., diameter) at the distal end of expandable member 20 (e.g., along all or part of distal section 20C of expandable member and/or at distal mouth 62) may be about 150 percent to about 300 percent wider than an inner dimension (e.g., diameter) of expandable member 20 near distal body portion 16B of elongated body 12.

Expandable member 20 can be configured to facilitate thrombus removal. In examples in which catheter 10 is used with an aspiration procedure (e.g., ADAPT), the size and shape of expandable member 20 may enable catheter 10 to better engage a thrombus by increasing the size of distal mouth 62 into which the thrombus may be received, increasing the total aspiration force exerted on the thrombus via a larger luminal area, and/or by distributing the aspiration forces over a greater portion of the thrombus rather than a localized area, thereby allowing the thrombus to be aspirated into catheter 10 more effectively. Expandable member 20 enables catheter 10 to maintain a relatively small diameter similar to that of elongated body 12 (e.g., within proximal body portion 16A) to facilitate navigability of catheter 10, while also enabling catheter 10 to exhibit improved engagement and suction force characteristics that may be attributed to having a large-diameter distal end 10B. In some examples, the presence of expandable member 20 may lead to improved revascularization success rates, such as due to the improved thrombus engagement and/or suction (e.g., to better pull the entirety of the thrombus into catheter 10 during aspiration) as described herein.

In addition, expandable member 20 can be configured to exhibit a relatively low longitudinally compressive stiffness, which can facilitate thrombus removal. For example, when combined with cyclical and/or pulsed aspiration, in which suction force applied to inner lumen 26 of catheter 10 is varied over time, the relatively low longitudinally compressive stiffness of expandable member 20 may enable the expandable member 20 to undergo "flutter"-type motion, in which expandable member 20 alternatingly contracts and expands in an axial direction (e.g., parallel to longitudinal axis 22), e.g., at a periodic frequency. This cyclical longitudinal contraction and expansion of expandable member 20 can in turn cause cyclical axial motion of the distal mouth 62 relative to the (stationary or relatively stationary) thrombus, which may facilitate dislodgment of the thrombus from vasculature. Additionally, as the expandable member 20 contracts longitudinally rather than radially in response to the application of cyclical aspiration, distal mouth 62 of expandable member 20 may remain more open and engaged with the thrombus, thereby further facilitating removal of the thrombus.

Expandable member 20 may be of any suitable length and diameter, which may be selected based on the target vessel or particular procedure being performed. For example, expandable member 20 may be made be long enough to fully engulf a thrombus (e.g., an average amount of thrombus material) and/or seal an outer surface of expandable member 20 against a vessel wall, but short enough to avoid excessive friction between the outer surface of expandable member 20 and an inner surface of an introducer sheath or an outer catheter. In some examples, expandable member 20 may be about 1 cm to about 25 cm long (to account for slight differences in length, such as due to manufacturing tolerances), measured in a direction parallel to longitudinal axis 22. For example, expandable member 20 may be about 1.5 cm, about 2.0 cm, or about 25 cm in length, such as from about 0.5 cm to about 3.0 cm.

As discussed above, in some examples, in the collapsed configuration, a distal section of expandable member 20 may have a cross-sectional dimension substantially equal to (e.g., equal to or nearly equal to) or less than the outer diameter of elongated body 12 proximate to expandable member 20. In some examples in which expandable member 20 defines a tube shape or a cylinder shape (having an open distal mouth 62) in an expanded configuration, expandable member 20 may define a substantially constant diameter (e.g., constant or nearly constant in the absence of forces compressing expandable member 20) along about 0.5 cm to about 3 cm, or 0.5 cm to about 2.5 cm of a length of expandable member 20, which can be a distal-most length in some examples. The length of expandable member 20 can be selected to be long enough to engulf a thrombus, but short enough to enable catheter 10 to be inserted into and/or withdrawn from a patient via an outer sheath. An expandable member that is too long may exert too much friction that interferes with movement of catheter 10 into or out of a patient via a sheath.

In some examples, in the expanded configuration, distal end 10B of expandable member 20 is larger than the outer diameter of elongated body 12, but smaller than the inner diameter of the target vasculature of the patient, such that expandable member 20 may be advanced through the vasculature of the patient while in the expanded configuration. Expandable member 20 may, for example, be configured to be in an expanded configuration within the vasculature of a patient without engaging with the vessel walls around an outer perimeter of expandable member 20, which may facilitate navigation of the expanded expandable member 20 through the vasculature. In some examples, distal end 10B of expandable member 20 may be about 105 percent to about 300 percent of the diameter of the proximal end of expandable member 20. In some examples, the expanded outer diameter or the cross-sectional dimension of expandable member 20 at distal end 10B may be about 105 percent to about 130 percent of the diameter of elongated body 12. As one illustrative example, catheter 10 may include an elongated body 12 defining an inner diameter of about 0.071 inches (about 0.180 cm) and an outer diameter of about 0.085 inches (about 0.216 cm), and expandable member 20 may define, in the expanded configuration, a maximum inner diameter of about 0.086 inches (about 0.218 cm) and a maximum outer diameter of about 0.096 inches (about 0.244 cm), corresponding to an expansion of the outer diameter of expandable member 20 to about 112 percent of the outer diameter of elongated body 12. In other examples, expandable member may expand to about 200 percent, 250 percent, 300 percent, or another larger percentage of the outer diameter or cross-sectional dimension of a portion of elongated body 12.

In some examples, the expandability of expandable member 20 at distal tip or portion 60 may allow the cross-sectional dimension of elongated body 12 within proximal body portion 16A to remain comparatively small. As described above, such a combination may allow catheter 10 to exhibit the improved navigability characteristics of a catheter body with a small diameter while still providing catheter 10 with the improved engagement and suction characteristics that may be attributed to having a large-diameter distal end 10B.

In some examples, an inner surface of expandable member 20 includes a surface treatment configured to promote at least one of mechanical or chemical engagement between the inner surface and the thrombus, and enable the thrombus to be pulled into lumen 26 of catheter 10 more effectively. For example, a coating may be applied to portions of the inner surface of expandable member 20 (e.g., the inner surface of inner liner 18, inner liner 19, or a portion of outer jacket 48 which does not surround an inner liner), where the coating has a relatively high clot affinity. Such affinity may be measured, for example, with a dynamic mechanical analyzer (DMA) equipped with a shear sandwich clamp. Examples of suitable coating materials to increase the affinity of the thrombus to expandable member 20 may include, for example, a thermoplastic elastomer such as ChronoPrene^{™} (AdvanSource Biomaterials, Wilmington, Massachusetts), ChronoPrene^{™} 5A, ChronoPrene^{™} 15A, a polyolefin elastomer such as ethylene-octene or ethylene-butene copolymer, for example, ENGAGE^{™} Polyolefin Elastomers (Dow Chemical Company, Midland, Michigan), ENGAGE^{™} 8107, 7367, 7270; or the like.

As another example, portions of the inner surface of expandable member 20 may be textured via etching or otherwise roughened (or rougher) in comparison to the outer surface of the expandable member 20 to better mechanically engage the thrombus. In some examples, an inner surface of expandable member 20 can include a polymer that is etched to promote mechanical thrombus engagement.

In some examples, thrombus engagement with expandable member 20 may be enhanced by delivering electrical energy to expandable member 20. For example, a source of electrical energy (e.g., an electrical signal generator) may deliver an electrical signal to expandable member 20 via one or more electrical conductors (not shown) electrically coupled to expandable member 20. The electrical energy may be positively charged to electrostatically engage a thrombus. Characteristics of the electrical energy may be adjusted to better engage the thrombus, such as polarity, or an amount or type of current delivered. For example, pulsed direct current may be employed, optionally with a non-square and/or non-negative waveform. The electrical conductors can extend through body inner lumen 26B of elongated body 12, can extend along an outer surface of elongated body 12, can be embedded in a wall of elongated body 12, or have any other suitable configuration.

Expandable member 20 may expand from a collapsed configuration to an expanded configuration using any suitable technique. In some examples, expandable member 20 may be balloon-expandable. For example, once elongated body 12 is positioned within the vessel of a patient adjacent a target treatment site, a balloon (not shown) may be introduced through lumen 26 of catheter 10 and inflated to radially expand expandable member 20 from a collapsed configuration to an expanded configuration. Once in the expanded configuration, expandable member 20 may maintain its shape to allow the balloon to be deflated and removed. Expandable member 20 may then be collapsed for removal from the vessel of the patient by, for example, pulling elongated body 12 or at least expandable member 20 into an outer sheath having an inner lumen with a diameter less than the outer diameter of an expanded expandable member 20. The outer sheath may apply an inward force to expandable member 20 as expandable member 20 is retracted proximally into the outer sheath.

In other examples, expandable member 20 may be configured to self-expand. For example, expandable support member 70 of expandable member 20 may be formed from a metal, and may include a shape-memory material such as Nitinol (and, optionally, additional material(s) or metal(s) such as radiopaque material(s) or metal(s)). Expandable support member 70 may be configured to self-expand to expand expandable member 20 radially outward (e.g., expand from a collapsed configuration to an expanded configuration). In some such examples as described further below, an outer sheath can be positioned over expandable member 20 to retain expandable member 20 in a collapsed configuration, e.g., during navigation of elongated body 12 to a target treatment site within the vasculature of a patient. Once at the target treatment site, the outer sheath can be retracted or elongated body 12 may be extended distally outward from the sheath to allow expandable member 20 to self-expand, e.g., via expandable support member 70. In other examples, catheter 10 may be navigated through vasculature with expandable member 20 in an expanded configuration.

In other examples, an electrical energy may be used to expand expandable member 20. For example, expandable member 20 (or a portion or a layer thereof) may be formed from a material or metal that bends or deflects in response to a current passed therethrough (or to heat generated as a result of such current). One such type of material is shape memory alloy actuator material, e.g., nitinol or Flexinol^{™} available from Dynalloy, Inc. of Irvine, California USA.

Hub 14 may be positioned at (e.g., proximal to or at least partially overlapping with) a proximal body portion 16A of elongated body 12. Proximal end 14A of hub 14 may define the catheter proximal end 10A of catheter 10 and may include an opening 30 aligned with body inner lumen 26B of elongated body 12, such that body inner lumen 26B of elongated body 12 may be accessed via opening 30 and, in some examples, closed via opening 30. For example, hub 14 may include a luer connector, a hemostasis valve, or another mechanism or combination of mechanisms for connecting hub 14 to another device such as a vacuum source for performing the aspiration techniques described herein. In some examples, proximal end 10A of catheter 10 can include another structure in addition to, or instead of, hub 14.

In some examples, first inner liner 18 and/or second inner liner 19 of elongated body 12 define at least a portion of inner lumen 26 (e.g., body inner lumen 26B) of catheter 10, where body inner lumen 26B defines a passageway through elongated body 12. In some examples, body inner lumen 26B extends within the entire length of first inner liner 18 (e.g., from proximal end 12A of elongated body 12 to the distal end 12B) and a portion of second inner liner 19. Body inner lumen 26B may be sized to receive a medical device (e.g., another catheter, a guidewire, an embolic protection device, a stent, or any combination thereof), a therapeutic agent, or the like. Elongated body 12, alone or with first inner liner 18, second inner liner 19 and/or other structures, may define a single inner lumen 26, or multiple inner lumens (e.g., two inner lumens or three inner lumens 26A to 26C) of catheter 10.

Body inner lumen 26B may be formed at least by first inner liner 18 and/or a portion of second inner liner 19, which may define the inner diameter of elongated body 12. The diameter of body inner lumen 26B (as measured in a direction perpendicular to a longitudinal axis 22 of elongated body 12) may vary based on the one or more procedures with which catheter 10 may be used. In some examples, the diameter of body inner lumen 26B of elongated body 12 may be substantially constant (e.g., constant or nearly constant) from proximal end 12A to distal end 12B or may taper (gradually or more step-wise) from a first inner diameter at proximal end 12A to a second, smaller inner diameter at distal end 12B. As described further below, an inner diameter of expandable member 20 may be larger than the inner diameter of elongated body 12 proximal to expandable member 20 while expandable member 20 is in an expanded configuration.

First inner liner 18 and/or second inner liner 19 may be coupled to respective portions of outer jacket 24 and/or outer jacket 48. In some examples, first inner liner 18 is adhered to or otherwise coupled to outer jacket 24 (e.g., with structural support member 28 being disposed between at least a portion of inner liner 18 and outer jacket 24). In some examples, inner liner 18 may also extend into expandable member 20 (e.g., a proximal portion of expandable member 20) such that a portion of inner liner 18 is adhered to or otherwise coupled to outer jacket 48. In some examples, inner liner 19 is adhered to or otherwise coupled to outer jacket 48 of expandable member 20, another inner surface of expandable member 20, or is otherwise coupled to expandable member 20. In some examples, a distal end (e.g., distalmost end) of first inner liner 18 abuts (e.g., contacts) a proximal end (e.g., proximalmost end) of second inner liner 19. In some examples, first inner liner 18 and second inner liner 19 are in a common radial layer of elongated body 12 (e.g., first inner liner 18 is not radially inward or radially outward from second inner liner 19 and/or second inner liner 19 is not radially inward or radially outward from first inner liner 18). In some examples, a distal end (e.g., a distalmost end) of outer jacket 24 abuts (e.g., contacts) a proximal end (e.g., a proximalmost end) of outer jacket 48. In some examples, the distal end of outer jacket 24 abuts outer jacket 48 at or approximately at the longitudinal location where first inner liner 18 abuts second inner liner 19.

First inner liner 18 and/or inner liner 19 may be formed using any suitable material, such as, but not limited to, polytetrafluoroethylene (PTFE), expanded PTFE (ePTFE, e.g., unidirectional ePTFE or bi-directional ePTFE), a fluoropolymer, perfluoroalkyoxy alkane (PFA), fluorinated ethylene propylene (FEP), polyolefin thermoplastic elastomer (TPE), other polyolefin elastomers, or any combination thereof. A unidirectional ePTFE may be stretched in one of the longitudinal or radial directions, and a bi-directional ePTFE may be stretched in both the longitudinal and radial directions. Other examples of materials from which inner liner 18 may be formed include, but are not limited to, Low Density Polyethylene (LDPE) (e.g., about 42D), a PTFE having a durometer of about 60D, High Density Polyethylene (HDPE), or any combination thereof. Some such polyolefin materials may have similar coefficients of friction as PTFE and may be conducive to processing.

In some examples, first inner liner 18 and second inner liner 19 include different materials. In some examples, first inner liner 18, which is proximal to second inner liner 19, includes PTFE and second inner liner 19 includes polyolefin TPE. The different materials of first inner liner 18 and second inner liner 19 may be selected because of/based on the different materials that first inner liner 18 and second inner liner 19 interact with. For example, as further described below, body outer jacket 24 (which is primarily radially outside of first inner liner 18) may include a different material than outer jacket 48 (which is primarily outside of second inner liner 19). However, in other examples, first inner liner 18 and second inner liner 19 include the same material. In some examples, second inner liner 19 is a distal extension (e.g., continuous extension) of first inner liner 18 (e.g., FIG. 3). In some examples, catheter 10 does not include first inner liner 18 and/or second inner liner 19.

In some examples, one or more portions of the inner surface of first inner liner 18, second inner liner 19, and/or a portion of the inner surface of expandable member 20 defining expandable member lumen 26C may be lubricious to facilitate the introduction and passage of a medical device (e.g., another catheter, a guide member, an embolic protection device, a stent, a thrombectomy device, or any combination thereof), a therapeutic agent, a thrombus, or the like, through body inner lumen 26B and/or expandable member lumen 26C. A lubricious first inner liner 18 and/or second inner liner 19 may also enable relatively easy tracking of elongated body 12 over a guide member (e.g., a guidewire or a microcatheter). In some examples, the material from which portions of first inner liner 18 and/or second inner liner 19 is formed may itself be lubricious (e.g., PTFE, polyolefin TPE, etc.). In addition to, or instead of, being formed from a lubricious material, in some examples, an inner surface of first inner liner 18 and/or second inner liner 19 is coated with a lubricious coating such as a hydrophilic coating.

Elongated body 12 includes one or more structural support members 28 positioned over at least a portion of first inner liner 18. Structural support member 28 is configured to increase the structural integrity of elongated body 12 while allowing elongated body 12 to remain relatively flexible. For example, structural support member 28 may be configured to help elongated body 12 substantially maintain its cross-sectional shape (e.g., circular or nearly circular) or at least help prevent elongated body 12 from buckling or kinking as it is navigated through tortuous anatomy. Additionally, or alternatively, structural support member 28, together with first inner liner 18, and outer jacket 24, may help distribute both pushing and rotational forces along a length of elongated body 12, which may help prevent kinking of elongated body 12 upon rotation of body 12 or help prevent buckling of body 12 upon application of a pushing force to elongated body 12. As a result, a clinician may apply pushing forces, rotational forces, or both, to the proximal portion of elongated body 12, and such forces may cause a distal portion of elongated body 12 to advance distally, rotate, or both, respectively.

Structural support member 28 may include one or more tubular braided structures, one or more coil members defining a plurality of turns, e.g., in the shape of a helix, or a combination of one or more braided structures and one or more coil members. Thus, although the examples of the disclosure primarily describe structural support member 28 as a coil, in other examples, catheter 10 may include a braided structure instead of a coil, a braided structure in addition to a coil, or a combination that includes one or more of each structure. As one example, a proximal portion of structural support member 28 may include a braided structure and a distal portion of structural support member 28 may include a coil member.

Structural support member 28 can be made from any suitable material, such as, but not limited to, a metal (e.g., a nickel titanium alloy (e.g., Nitinol), stainless steel, tungsten, titanium, gold, platinum, palladium, tantalum, silver, or a nickel-chromium alloy, a cobalt-chromium alloy, or the like), a polymer, a fiber, or any combination thereof. In some examples, structural support member 28 may include one or more metal wires braided or coiled around inner liner 18. The metal wires may include round wires, flat-round wires, flat wires, or any combination thereof. In other examples, structural support member 28 may include a spiral-cut hypotube that is positioned over inner liner 18.

As shown in the example of FIG. 2, structural support member 28 extends longitudinally along a portion of elongated body 12 and longitudinally overlaps with a portion of expandable member 20 (e.g., overlaps with a portion of proximal section 20A of expandable member 20). In some examples, a distal end of structural support member 28 extends distally of a proximal end of expandable member 20 and/or a proximal end of expandable support member 70. In examples where structural support member 28 longitudinally overlaps with expandable support member 70, expandable support member 70 may be radially outside of (as shown in FIG. 2), or radially inside of structural support member 28 (e.g., in the portion where expandable support member 70 overlaps with structural support member 28). However, in some examples, structural support member 28 is positioned proximal (e.g., entirely proximal to, or abutting) to expandable member 20 and/or expandable support member 70. In some examples, the distal end of structural support member 28 may abut (e.g., contact) the proximal end of expandable member 20 and/or expandable support member 70 and may be coupled to expandable member 20 and/or expandable support member 70 (e.g., mechanically coupled or bonded with adhesive, or welded). In other examples, expandable support member 70 may not be coupled to structural support member 28 or may not be in direct contact (e.g., abutting contact) with structural support member 28, although expandable support member 70 and structural support member 28 may be in the same radial layer of elongated body 12 (and/or have the same inner diameter and/or outer diameter where structural support member 28 and expandable member 20 meet or come closest to each other in the longitudinal direction). For example, the distal end of structural support member 28 may be adjacent to the proximal end of expandable member 20 and/or expandable support member 70 but separated by a small gap. In such examples where structural support member 28 and expandable support member 70 do not longitudinally overlap, structural support member 28 and expandable support member 70 may be in the same radial layer.

In some examples, structural support member 28 may be coupled, adhered, or mechanically connected to at least a portion of an outer surface of inner liner 18. For example, structural support member 28 may be positioned over inner liner 18 and secured in place (e.g., fixed) relative to inner liner 18 by outer jacket 24 using a melt-reflow/heat shrink process, via adhesives or other suitable technique.

Additionally or alternatively, structural support member 28 may be secured to inner liner 18 with the assistance of a support layer (not shown) that helps adhere structural support member 28 to one or both of inner liner 18 and outer jacket 24. The support layer may include a thermoplastic material or a thermoset material, such as a thermoset polymer or a thermoset adhesive that bonds to inner liner 18, outer jacket 24, or both. In some cases, the material forming the support layer may have elastic properties, such that there may be a tendency for the support layer to return to a resting position. In some examples, the support layer is positioned over the entire length of structural support member 28 and inner liner 18. In other examples, the support layer is only positioned over a part of the length of structural support member 28 and inner liner 18.

Elongated body 12 can also include outer jacket 24 positioned over structural support member 28 and inner liner 18, the structural support member 28 being positioned between portions of inner liner 18 and outer jacket 24. In some examples, outer jacket 24 may be positioned around structural support member 28 such that outer jacket 24 covers at least a part or all of both inner liner 18 and structural support member 28. Outer jacket 24, together with inner liner 18 and structural support member 28, may be configured to define elongated body 12 having the desired structural characteristics (e.g., flexibility, kink resistance, torque responsiveness, structural integrity, pushability, and column strength, which may be a measure of a maximum compressive load that can be applied to elongated body 12 without taking a permanent set).

For example, outer jacket 24 may have stiffness characteristics that contribute to the desired stiffness profile of elongated body 12.

In some examples, outer jacket 24 may be formed to have a stiffness that decreases from a proximal end 12A of elongated body 12 toward distal end 12B. The lowered stiffness of outer jacket 24 within the distal body portion 16B of elongated body 12 may improve the flexibility and navigability of catheter 10 through tortious vasculature of the patient, while the relatively higher stiffness of outer jacket 24 within the proximal body portion 16A of catheter 10 may provide better pushability or kink resistance. In some examples, outer jacket 24 may be formed from two or more different materials with different mechanical properties that enable outer jacket 24 to exhibit the desired stiffness characteristics. In some examples, first outer jacket 24 may define a stiffness that is greater than the stiffness of second outer jacket 48 of expandable member 20.

In some examples, outer jacket 24 may be formed using any suitable material including, but are not limited to, polymers, such as a polyether block amide (e.g., PEBAX^{®}, commercially available from Arkema Group of Colombes, France), an aliphatic polyamide (e.g., Grilamid^{®}, commercially available from EMS-Chemie of Sumter, South Carolina), another thermoplastic elastomer (e.g., a thermoplastic, elastomeric polymer configured to accommodate radial expansion of expandable member 20), polyurethanes, polyamides (e.g., Nylon-12), or other thermoplastic material, or combinations thereof.

Outer jacket 24 may be heat shrunk around structural support member 28 and, in some examples, at least a portion (e.g., a proximal portion) of expandable support member 70 to secure structural support member 28 and expandable support member 70 in the same radial layer. In some examples, during the heat shrinking of outer jacket 24 around structural support member 28, the material of outer jacket 24 may flow into at least some of the inner spacings or gaps (e.g., gaps between the adjacent turns of the coils, or between the struts or braids) within structural support member 28 or expandable support member 70 such that portions of outer jacket 24, structural support member 28, and/or expandable support member 70 form a laminated structure.

In some examples, at least a portion of an outer surface of outer jacket 24 and/or expandable member 20 includes one or more coatings, such as, but not limited to, an anti-thrombogenic coating, which may help reduce the formation of thrombi in vitro, an anti-microbial coating, and/or a lubricating coating. In some examples, the lubricating coating may be configured to reduce static friction or kinetic friction between elongated body 12 and tissue of the patient as elongated body 12 is advanced through the vasculature. In addition, or instead, in some examples, the lubricating coating may be configured to reduce static or kinetic friction between elongated body 12 and another catheter through which elongated body 12 may be inserted. The lubricating coating can be, for example, a hydrophilic coating. In some examples, the entire working length of elongated body 12 (from distal end 14B of hub 14 to the distal end of outer jacket 24) may be coated with the hydrophilic coating. In other examples, only a portion of the working length of elongated body 12 coated with the hydrophilic coating (e.g., about the distalmost 40 cm of catheter 10). This may provide a length of elongated body 12 distal from distal end 14B of hub 14 with which the clinician may grip elongated body 12, e.g., to rotate elongated body 12, pull elongated body 12 when removing elongated body 12 from the patient, or push elongated body 12 through vasculature.

Although a coating or another material may be applied over the outer surface of outer jacket 24, outer jacket 24 may still substantially define shape and size of the outer surface of elongated body 12. In some examples, the outer diameter of elongated body 12 may be substantially constant (e.g., constant or nearly constant) along the length of elongated body 12. In other examples, the outer diameter of elongated body 12 may taper from the first outer diameter within proximal body portion 16A of elongated body 12 to a second outer diameter at a point proximate to the proximal end of expandable member 20.

In some examples, expandable support member 70 is mechanically coupled to structural support member 28 and/or layered between (at least in a proximal portion of the expandable member 20) inner liner 18 and outer jacket 24. For example, expandable support member 70 and structural support member 28 can be formed independently of one another, and the proximal end or proximal portion of expandable support member 70 can be coupled to the distal end or distal portion of structural support member 28. In some examples, expandable support member 70 and structural support member 28 may be joined via welding, brazing, soldering, adhesives, epoxy, or other suitable technique. In some examples, expandable member 20 may be welded, soldered, bonded, or hooked to structural support member 28. In some examples, expandable support member 70 may be bonded (e.g., glued), hooked (e.g., mechanically interlocked), or coupled to structural support member 28 using other means.

In some examples, structural support member 28 and expandable support member 70 may be integrally formed. In some such examples, at least a proximal portion of expandable support member 70 (e.g., corresponding to a portion of proximal section 20A of expandable member 20) and structural support member 28 form the same radial layer of catheter 10, or in other words, are radially equidistant from central longitudinal axis 22. For example, structural support member 28 may include a plurality of wires (e.g., coils or braids) that are subsequently woven to form expandable support member 70, such that the manufacture may not necessarily require welding or other assembly or connection of expandable member 20 to structural support member 28.

Additionally, or alternatively, expandable support member 70 may be at least partially secured to structural support member 28 via inner liner 18 and/or outer jacket 24. For example, expandable support member 70 may not be directly coupled to structural support member 28. In an example, a proximal portion of expandable support member 70 may be positioned adjacent to (e.g., to be overlapping with) a portion (e.g., a distal portion) of structural support member 28 over inner liner 18, and outer jacket 24 may be positioned over expandable support member 70 and structural support member 28. Outer jacket 24 may be heat shrunk over the two members such that outer jacket 24 secures both expandable support member 70 and structural support member 28 in place relative to inner liner 18. In such examples, at least a portion of expandable support member 70 may be positioned at least partially between inner liner 18 and outer jacket 24.

In some examples, first inner liner 18 or second inner liner 19 does not extend over the entire longitudinal length of expandable member 20. For example, expandable member 20 may include second inner liner 19 (or a distal portion of inner liner 18 in examples where second inner liner 19 is not present) extending over only part of the length of expandable member 20 leaving portions of expandable member 20 exposed to expandable member lumen 26C. The exposed portions of expandable member 20 may provide better engagement with a thrombus and/or prevent distal migration of thrombus from catheter 10 due to the texture of expandable member 20 or direct electrostatic engagement with expandable member 20.

In some examples, both first inner liner 18 and second inner liner 19 terminate proximal to a distal end of expandable member 20. However, in some examples, at least one of first inner liner 18 or second inner liner 19 extend to a distalmost end of expandable member 20.

As described above, outer jacket 48 may include a polymer compounded with a radiographic material 72, which may enable outer jacket 48 (as well as expandable member 20 as a whole) to maintain relative flexibility while also being visible under certain medical imaging techniques. In some examples, radiographic material 72 includes one or more of tungsten, tungsten carbide, gadolinium, bismuth compounds (e.g., bismuth subcarbonate), tantalum, or barium sulfate, another suitable radiographic filler, or any combination thereof in any suitable proportions.

Outer jacket 48 of expandable member 20 may include a suitable polymer material or combination of polymer materials loaded with an amount of radiographic material such that outer jacket 48 resists degradation (e.g., via hydrolysis) while maintaining enough radiopacity for viewing under a suitable medical imaging technique. Because loading polymers with radiographic materials (e.g., tungsten) may hasten degradation (e.g., via hydrolysis), selecting a suitable polymer to resist degradation while being loaded with a radiopaque material may enable such materials to be incorporated into catheters to be used during medical procedures. In some examples, outer jacket 48 includes a Styrenic Block Copolymer (SBC), such as Styrene-Ethylene-Butylene-Styrene (SEBS) and/or hydrogenated (styrene-isoprene-styrene) copolymer (SIS). As compared to other polymers traditionally used in catheters (e.g., such as PEBAX, VESTAMID, etc.), SBCs may resist degradation (e.g., via hydrolysis) when loaded with radiographic materials while also facilitating the ability to tune and/or select the hardness for various catheter components. The properties of outer jacket 48 (such as the SBCs) may be tuned by adjusting the ratios of molecular weights and/or ratios of different polymer blocks. SBCs loaded with radiographic materials may exhibit a longer shelf life, as compared to other polymers loaded with radiographic materials. Further, SBCs loaded with radiographic materials may be able resist degradation during multiple heating cycles (e.g., extrusion, reflow, etc.), which may further catalyze degradation as compared to other polymers. For example, unlike other polymers such as nylon, the backbone of SBCs that include carbon-carbon bonds are less-reactive or non-reactive via hydrolysis, unless subjected to extreme temperatures (e.g., pyrolysis). Additionally, SBCs can be formulated to not include pendant functional groups, and are therefore can be considered a relative inert polymer. Further, SBCs can be formulated to be hydrogenated (no unsaturated double bonds), which may be a factor in other polymers degrading over time (e.g., yellowing of polymers, crosslinking, or chain scission).

The polymer (e.g., SBC) used for outer jacket 48 may include a suitable durometer or shore A hardness to enable expandable member 20 to maintain sufficient flexibility and/or compressibility, which may facilitate thrombus removal as discussed above. In some examples, the SBC used for outer jacket 48 has a shore A hardness of about 20A to about 80A hardness, such as about 30A hardness (e.g., about 30A hardness to account for slight variations, including manufacturing tolerances). In some examples, distal tip jacket 49 may additionally include an SBC (e.g., SEBS), e.g., to facilitate bonding to outer jacket 48. As discussed above, distal tip jacket 49 may include a stiffer material than outer jacket 48 (e.g., distal tip jacket 49 may include SEBS with a shore A hardness of 80A or about 80A). The stiffer material of distal jacket 49 as compared to outer jacket 48 may facilitate encapsulation of the loose wire ends of expandable support member 70. Other polymers that resist degradation when loaded with radiographic material may also be used for outer jacket 48 and/or distal outer jacket, including, but not limited to, polyolefins.

In some examples, the amount of radiographic material 72 loaded into the polymer for outer jacket 48 may be selected to maintain manufacturability and reduce a likelihood of degradation while maintaining enough radiopacity to enable a clinician to view expandable member 20 under a suitable medical imaging technique (e.g., x-ray, fluoroscopy, etc.). In some examples, the loading of radiographic material (e.g., tungsten) into the polymer of outer jacket 48 is about 50% to about 90% (inclusive), such as about 70% to about 80% by weight (inclusive) (e.g., to account for slight differences, such as due to manufacturing tolerances) in a polymer (e.g., an SBC), which equates to about 9% to about 15% by volume. In some examples, the loading of radiographic material (e.g., tungsten) into the polymer of outer jacket 48 is 74% or about 74% (e.g., to account for slight differences, such as due to manufacturing tolerances). Loading of radiographic material into a polymer jacket less than 70% be weight may not enable expandable member 20 to be visible enough under a suitable imaging technique, particularly in neurovascular applications because of many bony structures in the skull. However, loading of radiographic material into a polymer jacket less than 80% be weight may cause the polymer jacket to be difficult to manufacture (e.g., extrude) and/or reflow or may otherwise affect the mechanical properties of the polymer jacket.

In the examples described herein, the loading of radiographic material 72 is uniform or approximately uniform throughout outer jacket 48. However, in other examples, the loading of radiographic material 72 in outer jacket 48 is not uniform (e.g., a distalmost portion of outer jacket 48 may have a higher concentration of radiographic material 72 as compared to a more proximal portion of outer jacket 48). In some examples, a portion of outer jacket 48 over the taper of expandable support member 70 (e.g., corresponding to tapering section 20B or expandable member 20) may have a lower loading or no loading of radiographic material (e.g., to improve bonding and/or adhesion of polymer jacket 48 over the taper of expandable support member 70). The loading of radiographic material 72 into the polymer of outer jacket may occur by any suitable technique (e.g., melting the polymer and mixing in particles of radiographic material 72, etc.).

In some examples, the particle size of the radiographic material 72 may be selected to facilitate manufacturability, facilitate biocompatibility, and avoid leaching while maintaining visibility under a suitable medical imaging technique. A particle size may include a target (e.g., nominal) cross-sectional dimension (e.g., a diameter in examples with circular or near circular cross section) of radiographic particles. In some examples, a particle size (e.g., average diameter) of radiographic material 72 is about 100 nanometers or about 0.1 micrometers (e.g., to account for minor variations, such as due to manufacturing tolerances) to about 10 micrometers (e.g., to account for minor variations, such as due to manufacturing tolerances), such as 1 micrometer or about 1 micrometer (e.g., to account for minor variations, such as due to manufacturing tolerances). In some examples, compounding a polymer with a radiographic material having a particle size greater than 10 micrometers may be difficult to extrude.

In some examples, the polymer used in outer jacket 48 includes one or more functional group modifications to improve the adhesion and/or the ability of outer jacket 48 to bond to other materials (e.g., other materials in catheter 10, including braids, liners, coatings, and/or other layers or catheter sections). For example, modifying the polymer used in outer jacket 48 (e.g., the SBC) with one or more functional groups can improve adhesion of outer jacket 48 to body outer jacket 24, first inner liner 18, second inner liner 19, and/or one or more coatings (e.g., hydrophilic coating) applied to the inner or the outer surfaces of outer jacket 48 (e.g., hydrophilic coating). In some examples, the base polymer of outer jacket 48 (e.g., the SBC, including SEBS) is modified by grafting maleic anhydride (MA) (e.g., includes maleic anhydride functional groups). For example, SEBS modified with MA includes a MA-modified or MA-grafted SEBS (SEBS-g-MA). Modifying a base polymer (e.g., SEBS) with an additive (e.g., maleic anhydride) may improve adhesion of the base polymer to other materials (e.g., coatings) while maintaining biocompatibility. Other modifications include modifying a base polymer with an amine group or hydroxyl group. Further, other modifications to a base polymer to improve adhesion (e.g., of a hydrophilic coating) may include altering the hydrogenic properties of the based polymer.

In some examples, a mixture of unmodified SBC and a modified SBC (e.g., with a functional group) is formed to target a specific percentage of SBC with the functional group modification. For example, a mixture of SEBS and SEBS-g-MA can be used to target a specific percentage of SEBS-g-MA (e.g., 50% by volume or by weight SEBS-g-MA of the total SEBS).

While the examples described herein depict outer jacket 48 including a polymer with a uniform polymer flexibility and/or hardness, as well as a uniform loading of radiographic material 72 (e.g., uniform along a longitudinal axis 22 of distal portion 60 of catheter 10), outer jacket 48 may include sections having varied flexibility and/or varied loading concentrations of radiographic material.

In the example of FIG. 2, distal tip 49 is not loaded with a radiographic material. However, in other examples, distal tip jacket 49 may additionally be loaded with a radiographic material (which may be the same material as is loaded in outer jacket 48). In some examples, distal tip jacket 49 is loaded with a higher concentration of radiographic material as outer jacket 48. In some examples, distal tip jacket 49 is loaded with a lower concentration of radiographic material as outer jacket 48.

In some examples, after tubing is extruded from a polymer loaded with radiographic material, outer jacket 48 may be formed by reflowing the extruded tube over expandable support member 70 of expandable member 20. In some examples, (e.g., after reflowing outer jacket 48 over expandable support member 70), a hydrophilic coating may be applied on exterior surface of outer jacket 48 to provide lubricity for navigating the patient's vasculature.

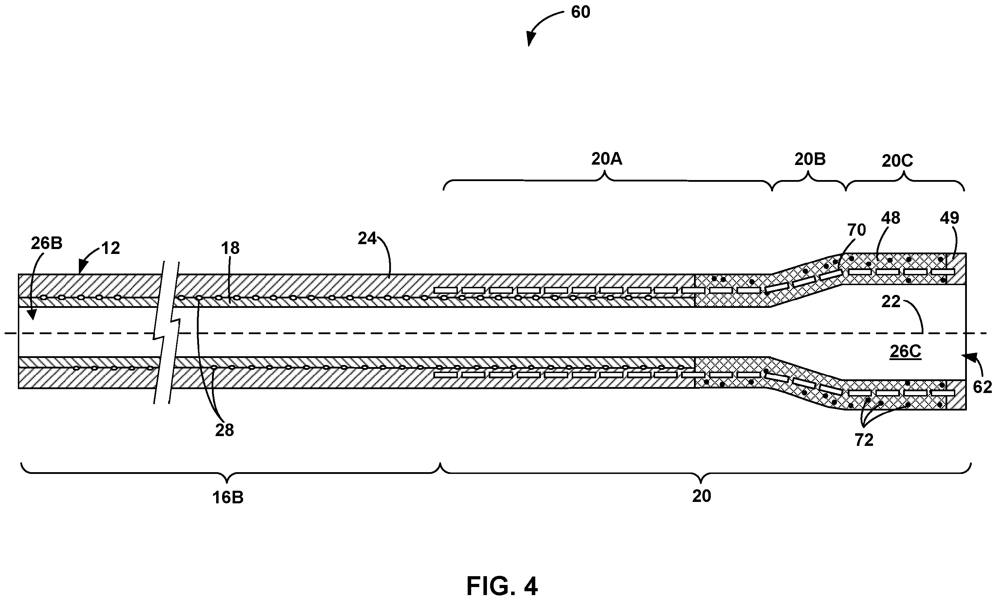

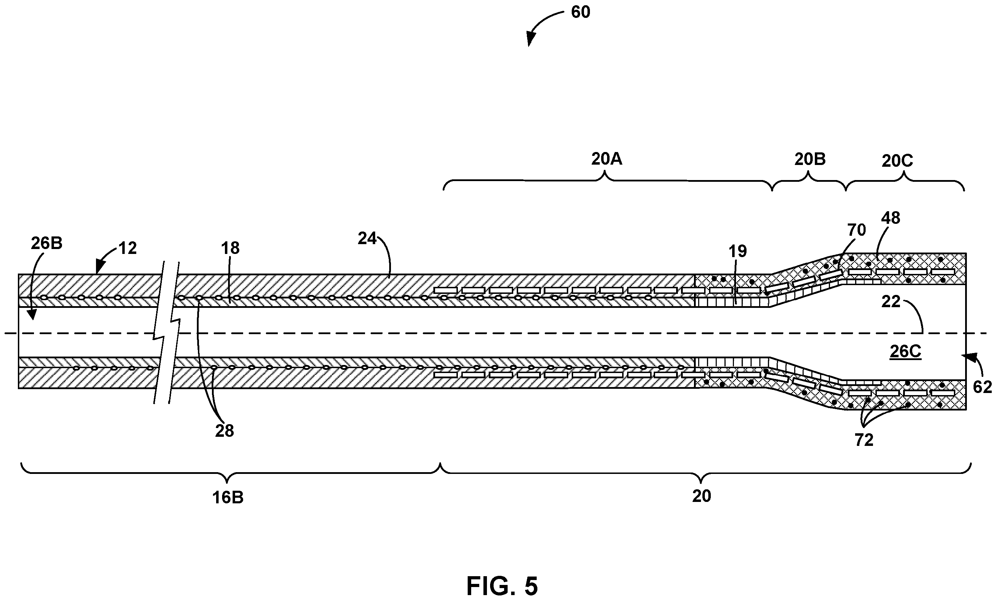

FIGS. 3 to 5 are conceptual cross-sectional views illustrating three examples of distal portion 60 of FIG. 1, where the cross section is taken through a center of distal portion 60 along a longitudinal axis 22. FIG. 3 depicts an example distal portion 60 where only one inner liner 18 is provided, which extends from elongated body 12 into expandable member 20. FIG. 4 depicts an example distal portion 60 where no liner is provided for the portion of expandable member 20 with the outer jacket 48, and inner liner 18 (which extends along elongated body 12) terminates at the distalmost end of the body outer jacket 24. FIG. 5 depicts an example depicts an example distal portion 60 where expandable member 20 does not include a separate distal tip distal to outer jacket 48. Any of the examples of FIGS. 3 to 5 can be combined in any permutation with each other, as well as with the examples of FIGS. 1 to 2.

In the example of FIG. 3, which is an example of distal portion 60 of FIG. 1, a second liner (e.g., inner liner 19 of FIG. 2) is not provided distal to inner liner 18, and inner liner 18 extends from a portion of elongated body 12 proximal of expandable member 20 to a portion of expandable member 20 distal of tapering section 20B. In this example, inner liner 18 may be configured to improve adhesion of outer jacket 48 to expandable support member 70 across the taper of expandable support member 70.

In the example of FIG. 4, which is an example of distal portion 60 of FIG. 1, an inner liner is not provided that is radially inside of outer jacket 48. In this example, inner liner 18 terminates proximally of outer jacket 48 and/or abuts (e.g., contacts) a proximal end outer jacket 48. In this example, outer jacket 48 encapsulates expandable support member 70 across the tapered section of expandable support member 70 (e.g., corresponding with tapering section 20B of expandable member 20). In examples where an inner liner is not provided over the tapered section of expandable support member 70, outer jacket 48 may have a lower loading or no loading of radiographic material across the tapered section of expandable support member 70. In the example of FIG. 4, lubricity may be provided to an inner surface of outer jacket 48 by applying a lubricious coating or other surface modification.

In the example of FIG. 5, which is an example of distal portion 60 of FIG. 1, elongated body 12 does not include a separate polymer jacket or tip member distal to outer jacket 48. In the example of FIG. 5, expandable support member 70 still terminates proximally of a distal end of outer jacket 48, or in other words, outer jacket 48 extends distally of a distalmost end of expandable support member 70. In this example, the portion of outer jacket 48 distal to expandable support member 70 may include a lower loading concentration or no loading of radiopaque material 72. However, in some examples, outer jacket 48 has a uniform loading of radiopaque material 72 extending to the distal tip of outer jacket 48, as well as the distal tip of elongated body 12.

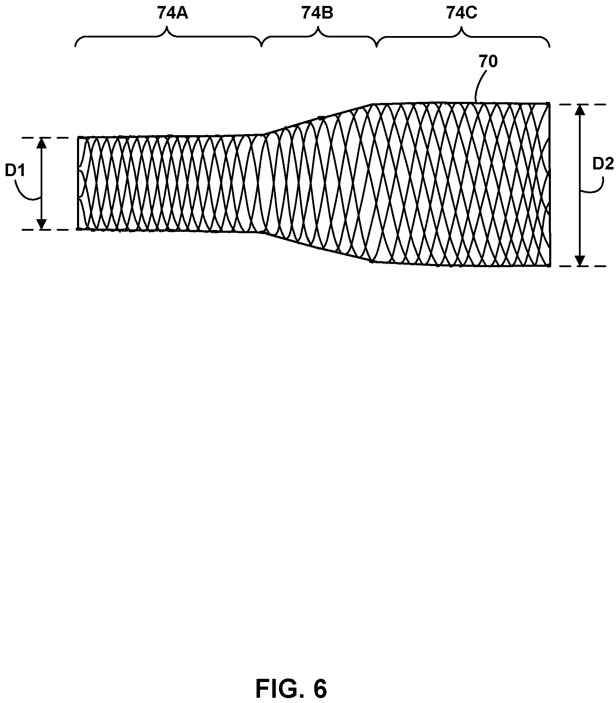

In some examples, such as the example shown in FIG. 6, expandable support member 70 includes braided structures with a tubular body comprising a plurality interwoven wires. In some examples, expandable support member 70 includes a proximal portion 74A (also referred to herein as proximal member portion 74A), a distal portion 74C (also referred to herein as distal member portion 74C), and a tapered portion 74B (also referred to herein as tapered member portion 74B) between proximal portion 74A and distal portion 74C. In some examples, proximal portion 74A defines a first elongated support member dimension D1 (e.g., an inner diameter D1 in examples in which elongated support member 70 defines a circular cross section) and distal portion 74C defines a second elongated support member dimension D2 (e.g., an inner diameter D2 in examples in which elongated support member 70 defines a circular cross section). In some examples, when expandable support member 70 is in a deployed (e.g., expanded) configuration, second elongated support member dimension D2 is greater than first elongated support member dimension D1. In some examples, tapered portion 74B defines a taper between first elongated support member dimension D1 of proximal portion 74A and second elongated support member dimension D2 of distal portion 74C.

In some examples, expandable support member 70 includes a self-expanding material (e.g., nitinol) and is configured to have sufficient axial and radial strength to resist collapse during a medical procedure (e.g., when a vacuum force is pulled or during a medical procedure). In this way, expandable support member 70 may provide structural support to expandable member 20 and a portion of elongated body 12 (e.g., a portion of distal portion 16B).

In some examples, expandable support member 70 is configured to be heat-set on a mandrel to establish the different diameters of proximal portion 74A, tapered portion 74B, and distal portion 74C. The diameters of proximal portion 74A, tapered portion 74B, and distal portion 74C of expandable support member 70 may be configured (e.g., during heat-setting) according to the desired diameters of respective sections proximal section 20A, tapering section 20B, distal section 20C of expandable member 20 as described above. For example, diameter D1 of proximal portion 74A may correspond to an inner diameter and/or outer diameter of proximal section 20A of expandable member 20. As another example, diameter D2 of distal portion 74C may correspond to an inner diameter/and or outer diameter of distal section 20C of expandable member 20. In some examples, as discussed above, the greater diameter D2 of distal portion 74C (and thus the expandable member 20) may enable engagement with a thrombus (e.g., clot or embolus).

The taper profile of tapered portion 74B or expandable support member 70 may defined during heat-set. Tapered portion 74B may taper from dimension D2 (e.g., diameter) at a distalmost end of tapered portion 74B to dimension D1 (e.g., diameter) at a proximal most end of tapered portion 74B. In some examples, tapered portion 74B defines a constant (e.g., linear) taper. In some examples, tapered portion 74B defines a changing (e.g., non-linear or curved) taper.

Expandable support member 70 may include a suitable braid configuration to provide radial and axial strength to prevent collapse of expandable member 20 while a suction force is pulled through expandable support member 70 or when expandable support member 70 is collapsed for retrieval. In some examples, expandable support member 70 includes a 16-wire braid with 1 over 1 braid geometry. In some examples, expandable support member 70 includes a braid density of about 100 picks per inch (PPI) (e.g., such as to account in minor variations, such as due to manufacturing tolerances). In some examples, expandable support member 70 includes a braid density of greater than 100 picks per inch (PPI). In some examples, expandable support member 70 includes a wire size of about 0.001 inches to about 0.003 inches (which is about 0.0254 mm to about 0.0762 mm).

In some examples, catheter 10 is introduced into the vasculature of a patient with the aid of an introducer sheath, which defines a pathway from an exterior access point into the vasculature (e.g., a radial artery or a femoral artery of the patient). For example, FIGS. 7A and 7B are conceptual cross-sectional side views of expandable member 20 of catheter 10 (e.g., FIGS. 1 and 2) being deployed with the aid of an introducer sheath 56. FIG. 7A illustrates expandable member 20 in a collapsed configuration within introducer sheath 56 positioned over expandable member 20. In the collapsed configuration, the expandable member 20 may be configured to have a low profile and be navigated through introducer sheath 56. Introducer sheath 56 may include a tubular body configured to receive catheter 10. FIG. 7B illustrates expandable member 20 in a deployed (e.g., expanded) configuration, when expandable member 20 is advanced distally of (e.g., at least partially of) introducer sheath 56.

In some examples, a clinician positions introducer sheath 56 from an incision site (e.g., a femoral access site or a radial access site) and into a patient's vasculature and then introduces catheter 10 into the vasculature through introducer sheath 56. In some examples, catheter 10 is introduced directly into the vasculature via introducer sheath 56. In examples in which expandable member 20 is self-expandable (e.g., via expandable support member 70), expandable member 20 is deployed into the expanded configuration (also referred to herein as a deployed configuration) upon advancement distally of a distal opening of introducer sheath 56 (as shown in FIG. 7B). In the expanded configuration, expandable member 20 is configured to engage a thrombus. In some examples, clinician may then navigate catheter 10 through the vasculature of the patient while expandable member 20 is already in the deployed configuration shown in FIG. 7B.

In other examples, catheter 10 may be positioned without an outer catheter that holds expandable member 20 in a collapsed configuration until the expandable member 20 reaches a target site within vasculature of a patient. In some of these examples, a clinician may introduce the outer catheter (in which catheter 10 is positioned) into the vasculature via introducer sheath 56.

Catheter 10 may be loaded into introducer sheath 56 directly or with the aid of an insertion tool (not shown). As described above with respect to FIGS. 1 and 2, an advantage of eliminating a more rigid solid metal marker band at the distal tip or distal portion 60 of catheter 10 is that expandable member 20 may be easily necked down or collapsed to fit within introducer sheath 56. In some examples, an insertion tool (also referred to as an "introducer tool" or a "compression tool") is configured to collapse expandable member 20 into a collapsed or delivery configuration and enable expandable member 20 to fit within an inner lumen of introducer sheath 56. A clinician may use introducer tool to insert expandable member 20 into introducer sheath 56, e.g., during a medical procedure or in preparation for the medical procedure.

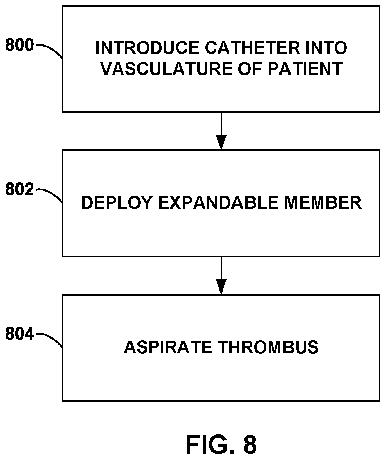

FIG. 8 is a flow diagram of an example method of aspiration using catheter 10 of FIGS. 1 and 2, but is applicable to any of the catheters described in this disclosure (including the examples of distal portion 60 described in FIGS. 3 to 5). The technique of FIG. 8 includes inserting catheter 10 into vasculature of the patient (800), deploying expandable member 20 to expand expandable member 20 in the vasculature of the patient (802), and aspirating a thrombus (804). In some examples, the techniques described herein include removing catheter 10 from the vasculature of the patient once the procedure is complete. Throughout the techniques of FIG. 8, a clinician may observe a radiopaque material of expandable member 20 via fluoroscopic imaging (or another suitable medical imaging technique) to improve surgical performance and patient outcomes.

A clinician may observe, using a suitable medical imaging technique (e.g., fluoroscopic imaging), expandable member 20 while distally advancing distal tip or distal portion 60 of catheter 10 toward a target site. In some examples, inserting catheter 10 into vasculature of a patient (800) includes initially introducing a guidewire, guide catheter, or another guide member into the vasculature of the patient to a target treatment site. Elongated body 12 may then be introduced over the guidewire and advanced to the target treatment site. Additionally, or alternatively, catheter 10 may be introduced into vasculature of a patient with the aid of a guide catheter. For example, the guide catheter may be initially introduced into vasculature of a patient and positioned adjacent a target treatment site. Catheter 10 may then be introduced through an inner lumen of the guide catheter.

Once within the vasculature, expandable member 20 may be deployed into the vasculature (802). In some examples, expandable member 20 may be self-expanding and may expand without the aid of any additional expansion mechanisms once released from introducer sheath 56 or another outer sheath. Additionally, or alternatively, expandable member 20 may be expanded using a balloon. In other examples, expandable member may be expanded by applying electrical energy to expandable member 20. For example, expandable member 20 (or a portion or layer thereof) may be constructed using a shape memory alloy actuator material.

The technique of FIG. 8 also includes applying a suction force to inner lumen 26 of catheter 10 to remove a thrombus from the vasculature (804). For example, once distal tip or distal portion 60 of catheter 10 is positioned proximate to a thrombus, a clinician may actuate a suction source to apply a suction force to lumen 26. The suction source can comprise a pump, such as a direct-acting pump (e.g., a peristaltic pump, or a lobe, vane, gear, or piston pump, or other suitable pumps of this type) or an indirect-acting pump (e.g., a vacuum pump, which creates a partial vacuum in an evacuation volume fluidically coupled to the liquid to be displaced). Due to the radiopacity of expandable member 20, a clinician may observe or determine, using a suitable medical imaging technique (e.g., fluoroscopic imaging), a longitudinal or axial contraction of expandable member 20 during the aspiration, as well as a shape of expandable member 20, which may indicate that the expandable member 20 is in engaged with the thrombus.

In some examples, the suction force applied to inner lumen 26 of catheter 10 is varied over time, referred to herein as cyclical aspiration. As discussed above, during this cyclical aspiration, expandable member 20 may axially compress and expand in response to the varying suction force.

Catheter 10 may be repositioned or removed from the vasculature once the aspiration procedure is complete. In some examples, catheter 10 is retracted into a sheath (e.g., introducer sheath 56 of FIGS. 7A and 7B) for repositioning or removal from the vasculature (e.g., to collapse expandable member 20 back to the collapsed configuration). In some examples, after retraction, catheter 10 is moved to a different vasculature site to aspirate a different thrombus. In some examples, after the aspiration procedure is complete, catheter 10 may be removed from an incision site.

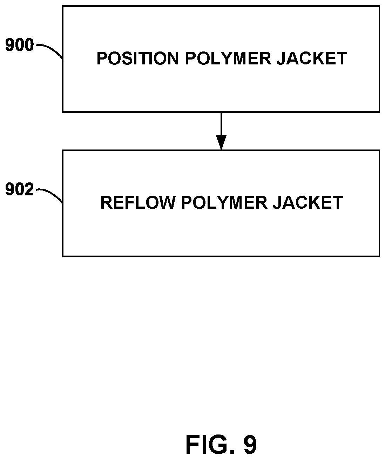

FIG. 9 is a flow diagram of an example method of forming catheter 10 of FIGS. 1 and 2, but is applicable to any of the catheters described in this disclosure (including the examples of distal portion 60 described in FIGS. 3 to 5). The technique of FIG. 9 includes positioning polymer jacket 48 over a portion of expandable support member 70 (900) and reflowing polymer jacket 48 onto catheter 10 (902). In some examples, prior to positioning polymer jacket over a portion of expandable support member 70, the method further includes mixing a polymer with a radiographic material (e.g., radiographic material 72) to form a radiographic polymer compound and extruding the polymer jacket 48 (e.g., a polymer tube) from the radiographic polymer compound.

In some examples, positioning polymer jacket 48 over a portion of expandable support member 70 (900) includes positioning the extruded polymer tube forming polymer jacket 48 over a portion of expandable support member 70 (e.g., a distal portion of expandable support member 70). After positing polymer jacket 48 over a portion of expandable support member 70, a proximal end of polymer jacket 48 may abut a distal end of body polymer jacket 24. In some examples, as discussed above, polymer jacket 48 includes a different material than body polymer jacket 24. A mandrel defining a taper may be provided in (e.g., radially inward from) expandable support member 70 and polymer jacket 48 before reflowing.

In some examples, reflowing polymer jacket 48 onto catheter 10 (902) includes providing heat to polymer jacket 48 to cause the polymer to melt and encapsulate a portion of expandable support member 70. Additionally, reflowing may case polymer jacket 48 to bond to outer jacket 24. Other polymer jackets (e.g., distal tip outer jacket 49) may further be reflowed.

The examples described herein may be combined in any permutation or combination.

Example 1: A catheter includes an elongated body comprising a distal body portion and defining a body inner lumen; and an expandable member coupled to and extending from the distal body portion and comprising an expandable support member and a polymer jacket, wherein the expandable member defines an expandable member inner lumen, wherein the expandable member inner lumen is in fluid communication with the body inner lumen, wherein the expandable support member comprises a tubular body and is configured to self-expand to expand the expandable member inner lumen radially outward, and wherein the polymer jacket at least partially overlays the expandable support member and comprises a polymer loaded with a radiographic material, the radiographic material configured to be visible via medical imaging.

Example 2: The catheter of example 1, wherein the polymer jacket comprises a Styrenic Block Copolymer (SBC).

Example 3: The catheter of example 2, wherein the SBC comprises at least one of Styrene-Ethylene-Butylene-Styrene (SEBS) or hydrogenated (styrene-isoprene-styrene) (SIS).

Example 4: The catheter of any of examples 2 or 3, wherein the SBC includes maleic anhydride functional groups.

Example 5: The catheter of any of examples 1 through 4, wherein the radiographic material comprises at least one of tungsten, tungsten carbide, gadolinium, barium sulfate, bismuth or tantalum.

Example 6: The catheter of any of examples 1 through 5, wherein the polymer jacket comprises about 50% to 90% radiographic material by weight.

Example 7. The catheter of any of examples 1 through 6, wherein the radiographic material defines a particle size of about 100 nanometers to about 10 micrometers.

Example 8. The catheter of any of examples 1 through 7, wherein the polymer of the polymer jacket comprises a Shore A hardness of about 30A to about 80A.

Example 9. The catheter of any of examples 1 through 8, wherein the elongated body comprises a body polymer jacket coupled to the polymer jacket, and wherein the body polymer jacket comprises a body polymer different than the polymer of the polymer jacket.

Example 10. The catheter of any of examples 1 through 9, wherein the elongated body comprises a distal tip polymer jacket distal to the polymer jacket, the distal tip polymer jacket configured to encapsulate a distal end of the expandable support member.

Example 11. The catheter of example 10, wherein the distal tip polymer jacket comprises a distal tip polymer, wherein the distal tip polymer is harder than the polymer of the polymer jacket such that the distal end of the expandable support member is not exposed to vasculature of a patient.

Example 12. The catheter of any of example 1 through 11, wherein the expandable support member includes a tapered portion defining a taper between a first elongated support member dimension and a second elongated support member dimension, and wherein the elongated body further comprises: an inner liner located radially inward to at least a portion of the polymer jacket, wherein the inner liner extends along at least a portion of the tapered portion of the expandable support member.

Example 13. The catheter of example 12, wherein the inner liner is a first inner liner comprising a first liner material, and wherein the elongated body further comprises: a second inner liner located radially inward to at least a portion of the polymer jacket and comprising a second liner material, wherein the second inner liner is proximal to first inner liner such that the second inner liner abuts the first inner liner, wherein the second inner liner and the first inner layer are in a common radial layer of the elongated body, and wherein the first liner material is different than the second liner material.

Example 14. The catheter of any of examples 1 through 13, wherein the expandable member is configured to expand radially outward via the expandable support member from a collapsed configuration to an expanded configuration, wherein when the expandable member is in the collapsed configuration, the expandable member is configured to navigated through an introducer sheath, and wherein when the expandable member is in the expanded configuration, the expandable member is radially expanded relative to the collapsed configuration and configured to engage a thrombus.

Example 15. The catheter of example 14, wherein the expandable support member comprises a proximal member portion defining a first inner diameter and a distal member portion defining a second inner diameter, wherein in the expanded configuration, the second inner diameter is greater than the first inner diameter such that the expandable member is configured to engage a thrombus.

Example 16. The catheter of any of examples 1 through 15, wherein the elongated body further comprises: a structural support member comprising a coil and configured to maintain a cross-sectional shape of the elongate body, wherein the structural support member longitudinally overlaps with the expandable support member.

Example 17. The catheter of any of examples 1 through 16, wherein the polymer jacket fully overlays the expandable support member.

Example 18. The catheter of any of examples 1 through 17, wherein the elongated body does not include a solid metal radiopaque marker band.

Example 19. A catheter comprising: an elongated body comprising a distal body portion and defining a body inner lumen; and a polymer jacket located at the distal body portion, the polymer jacket comprising a polymer loaded with a radiographic material, the radiographic material configured to be visible via medical imaging, wherein the polymer comprises a Styrenic Block Copolymer (SBC).

Example 20. The catheter of example 19, wherein the SBC comprises at least one of Styrene-Ethylene-Butylene-Styrene (SEBS) or hydrogenated (styrene-isoprene-styrene) (SIS).

Example 21. The catheter of any of examples 19 or 20, wherein the SBC includes maleic anhydride functional groups.

Example 22. The catheter of any of examples 19 through 21, wherein the radiographic material includes at least one of tungsten, tungsten carbide, gadolinium, barium sulfate, bismuth or tantalum.

Example 23. The catheter of any of examples 19 through 22, wherein the polymer jacket comprises about 50% to 90% radiographic material by weight.

Example 24. The catheter of any of examples 19 through 23, wherein the radiographic material comprises a particle size of about 100 nanometers to 10 micrometers.

Example 25. The catheter of any of examples 19 through 24, wherein the polymer of the polymer jacket comprises a Shore A hardness of about 30A to 80A.

Example 26. The catheter of any of examples 19 through 25, wherein the elongated body comprises a body polymer jacket proximal to the polymer jacket, wherein the body polymer jacket comprises a body polymer different than the polymer of the polymer jacket.

Example 27. The catheter of any of examples 19 through 26, wherein the elongated body comprises a distal tip polymer jacket distal to the polymer jacket, wherein the distal tip polymer jacket comprises a distal tip polymer, wherein the distal tip polymer is harder than the polymer of the polymer jacket.

Example 28. A method comprising: positioning a polymer jacket including a radiographic material over a portion of an expandable support member of a catheter; and reflowing the polymer jacket onto the catheter, wherein when assembled, the catheter comprises: an elongated body comprising a distal body portion and defining a body inner lumen; and an expandable member coupled to and extending from the distal body portion and comprising the expandable support member and the polymer jacket, wherein the expandable member defines an expandable member inner lumen, wherein the expandable member inner lumen is in fluid communication with the body inner lumen, wherein the expandable support member comprises a tubular body and is configured to self-expand to expand the expandable member inner lumen radially outward, and wherein the polymer jacket at least partially overlays the expandable support member and comprises a polymer loaded with a radiographic material, the radiographic material configured to be visible via medical imaging.

Example 29. The method of example 28, wherein, after positioning the polymer jacket over the portion of the expandable support member, the polymer jacket abuts a body polymer jacket, wherein the body polymer jacket comprises a body polymer different than the polymer of the polymer jacket.

Example 30. The method of example 28, further comprising: mixing a polymer with the radiographic material to form a radiographic polymer compound; and extruding the polymer jacket from the radiographic polymer compound.

Various aspects of the disclosure have been described. These and other aspects are within the scope of the following claims.

## Claims

1. A catheter comprising:
an elongated body comprising a distal body portion and defining a body inner lumen; and
an expandable member coupled to and extending from the distal body portion and comprising an expandable support member and a polymer jacket, wherein the expandable member defines an expandable member inner lumen, wherein the expandable member inner lumen is in fluid communication with the body inner lumen, wherein the expandable support member comprises a tubular body and is configured to self-expand to expand the expandable member inner lumen radially outward, and wherein the polymer jacket at least partially overlays the expandable support member and comprises a polymer loaded with a radiographic material, the radiographic material configured to be visible via medical imaging.

2. The catheter of claim 1, wherein the polymer jacket comprises a Styrenic Block Copolymer (SBC), and more specifically wherein the SBC comprises at least one of Styrene-Ethylene-Butylene-Styrene (SEBS) or hydrogenated (styrene-isoprene-styrene) (SIS).

3. The catheter of any of claims 1 or 2, wherein the radiographic material comprises at least one of tungsten, tungsten carbide, gadolinium, barium sulfate, bismuth or tantalum.

4. The catheter of any of claims 1 through 3, wherein the elongated body comprises a body polymer jacket coupled to the polymer jacket, and wherein the body polymer jacket comprises a body polymer different than the polymer of the polymer jacket.

5. The catheter of any of claims 1 through 4, wherein the elongated body comprises a distal tip polymer jacket distal to the polymer jacket, the distal tip polymer jacket configured to encapsulate a distal end of the expandable support member.

6. The catheter of claim 5, wherein the distal tip polymer jacket comprises a distal tip polymer, wherein the distal tip polymer is harder than the polymer of the polymer jacket such that the distal end of the expandable support member is not exposed to vasculature of a patient.

7. The catheter of any of claims 1 through 6, wherein the expandable support member includes a tapered portion defining a taper between a first elongated support member dimension and a second elongated support member dimension, and
wherein the elongated body further comprises:
an inner liner located radially inward to at least a portion of the polymer jacket, wherein the inner liner extends along at least a portion of the tapered portion of the expandable support member.

8. The catheter of claim 7, wherein the inner liner is a first inner liner comprising a first liner material, and wherein the elongated body further comprises:
a second inner liner located radially inward to at least a portion of the polymer jacket and comprising a second liner material,
wherein the second inner liner is proximal to first inner liner such that the second inner liner abuts the first inner liner,
wherein the second inner liner and the first inner layer are in a common radial layer of the elongated body, and
wherein the first liner material is different than the second liner material.

9. The catheter of any of claims 1 through 8, wherein the expandable member is configured to expand radially outward via the expandable support member from a collapsed configuration to an expanded configuration, wherein when the expandable member is in the collapsed configuration, the expandable member is configured to navigated through an introducer sheath, and wherein when the expandable member is in the expanded configuration, the expandable member is radially expanded relative to the collapsed configuration and configured to engage a thrombus.

10. The catheter of claim 9, wherein the expandable support member comprises a proximal member portion defining a first inner diameter and a distal member portion defining a second inner diameter, wherein in the expanded configuration, the second inner diameter is greater than the first inner diameter such that the expandable member is configured to engage a thrombus.

11. The catheter of any of claims 1 through 10, wherein the elongated body further comprises:
a structural support member comprising a coil and configured to maintain a cross-sectional shape of the elongate body,
wherein the structural support member longitudinally overlaps with the expandable support member.

12. The catheter of any of claims 1 through 11, wherein the polymer jacket fully overlays the expandable support member.

13. A method comprising:
positioning a polymer jacket including a radiographic material over a portion of an expandable support member of a catheter; and
reflowing the polymer jacket onto the catheter, wherein when assembled, the catheter comprises:
an elongated body comprising a distal body portion and defining a body inner lumen; and
an expandable member coupled to and extending from the distal body portion and comprising the expandable support member and the polymer jacket, wherein the expandable member defines an expandable member inner lumen, wherein the expandable member inner lumen is in fluid communication with the body inner lumen, wherein the expandable support member comprises a tubular body and is configured to self-expand to expand the expandable member inner lumen radially outward, and wherein the polymer jacket at least partially overlays the expandable support member and comprises a polymer loaded with a radiographic material, the radiographic material configured to be visible via medical imaging.

14. The method of claim 13, wherein, after positioning the polymer jacket over the portion of the expandable support member, the polymer jacket abuts a body polymer jacket, wherein the body polymer jacket comprises a body polymer different than the polymer of the polymer jacket.

15. The method of claim 13, further comprising:
mixing a polymer with the radiographic material to form a radiographic polymer compound; and
extruding the polymer jacket from the radiographic polymer compound.
